# EUROPEAN PATENT APPLICATION

(11) **EP 1 900 375 A1**
(43) Date of publication of application: **19.03.2008**
(21) Application number: 06291427.0
(22) Date of filing: 08.09.2006
(51) Int. Cl.: A61K 38/17, A61K 38/45, A61K 31/70, A61K 31/35, A61K 39/395, G01N 33/00, A61P 35/00, A61P 37/06

(54) **Calreticulin for its use as a medication for the treatment of cancer in a mammal**

(71) Applicant: Institut Gustave Roussy, 94805 Villejuif Cédex (FR)
(72) Inventor: Obeid, Michel, 75014 Paris (FR); Kromer, Guido, 94805 Villejuif (FR); Zitvogel, Laurence, 94805 Villejuif (FR)
(74) Representative: Starck-Loudes, Anne-Caroline

(57) **Abstract**

The present invention concerns the calreticulin for its use as a medication for the treatment of a disease in a mammal, said medication inducing an increased location of calreticulin at the cellular surface.

Application for treating disease such as cancer.

SEQUENCE LISTING

<110>  INSTITUT GUSTAVE ROUSSY Centre de lutte contre le cancer

<120>  Calreticulin for its use as a medication for the treatment of a
       disease such as cancer in a mammal

<130>  B 52086 EP

<140>  EP 062914270
<141>  2006-09-08

<160>  1

<170>  PatentIn version 3.3

<210>  1
<211>  416
<212>  PRT
<213>  mouse

<400>  1

Met Leu Leu Ser Val Pro Leu Leu Leu Gly Leu Leu Gly Leu Ala Ala
1               5                   10                  15

Ala Asp Pro Ala Ile Tyr Phe Lys Glu Gln Phe Leu Asp Gly Asp Ala
            20                  25                  30

Trp Thr Asn Arg Trp Val Glu Ser Lys His Lys Ser Asp Phe Gly Lys
        35                  40                  45

Phe Val Leu Ser Ser Gly Lys Phe Tyr Gly Asp Leu Glu Lys Asp Lys
    50                  55                  60

Gly Leu Gln Thr Ser Gln Asp Ala Arg Phe Tyr Ala Leu Ser Ala Lys
65                  70                  75                  80

Phe Glu Pro Phe Ser Asn Lys Gly Gln Thr Leu Val Val Gln Phe Thr
                85                  90                  95

Val Lys His Glu Gln Asn Ile Asp Cys Gly Gly Gly Tyr Val Lys Leu
            100                 105                 110

Phe Pro Ser Gly Leu Asp Gln Lys Asp Met His Gly Asp Ser Glu Tyr
        115                 120                 125

Asn Ile Met Phe Gly Pro Asp Ile Cys Gly Pro Gly Thr Lys Lys Val
    130                 135                 140

His Val Ile Phe Asn Tyr Lys Gln Lys Asn Val Leu Ile Asn Lys Asp
145                 150                 155                 160

Ile Arg Cys Lys Asp Asp Glu Phe Thr His Leu Tyr Thr Leu Ile Val
                165                 170                 175

Arg Pro Asp Asn Thr Tyr Glu Val Lys Ile Asp Asn Ser Gln Val Glu
            180                 185                 190

Ser Gly Ser Leu Glu Asp Asp Trp Asp Phe Leu Pro Pro Lys Lys Ile
        195                 200                 205

Lys Asp Pro Asp Ala Ala Lys Pro Glu Asp Trp Asp Glu Arg Ala Lys
    210                 215                 220

Ile Asp Asp Pro Thr Asp Ser Lys Pro Glu Asp Trp Asp Lys Pro Glu
225                 230                 235                 240

His Ile Pro Asp Pro Asp Ala Lys Lys Pro Glu Asp Trp Asp Glu Glu
                245                 250                 255

Met Asp Gly Glu Trp Glu Pro Pro Val Ile Gln Asn Pro Glu Tyr Lys
            260                 265                 270

Gly Glu Trp Lys Pro Arg Gln Ile Asp Asn Pro Asp Tyr Lys Gly Thr
        275                 280                 285

Trp Ile His Pro Glu Ile Asp Asn Pro Glu Tyr Ser Pro Asp Ala Asn
    290                 295                 300

Ile Tyr Ala Tyr Asp Ser Phe Ala Val Leu Gly Leu Asp Leu Trp Gln
305                 310                 315                 320

Val Lys Ser Gly Thr Ile Phe Asp Asn Phe Leu Ile Thr Asn Asp Glu
                325                 330                 335

Ala Tyr Ala Glu Glu Phe Gly Asn Glu Thr Trp Gly Val Thr Lys Ala

340                 345                 350

Ala Glu Lys Gln Met Lys Asp Lys Gln Asp Glu Glu Gln Arg Leu Lys
        355                 360                 365

Glu Glu Glu Glu Asp Lys Lys Arg Lys Glu Glu Glu Glu Ala Glu Asp
    370                 375                 380

Lys Glu Asp Asp Asp Asp Arg Asp Glu Asp Glu Asp Glu Glu Asp Glu
385                 390                 395                 400

Lys Glu Glu Asp Glu Glu Glu Ser Pro Gly Gln Ala Lys Asp Glu Leu
                405                 410                 415

## Description

### Domain of the invention

The present invention relates to isolated and purified proteins, such as calreticulin, recombinant calreticulin, mimetics of calreticulin or molecules that induce calreticulin exposure at the cell surface for a novel use as a medication in the treatment of diseases such as cancer. Moreover, the invention deals with a new method of treating diseases such as cancers comprising an induction of an immunogenic apoptosis and increased efficiency of chemotherapy. The invention also concerns a method of detection of the calreticulin protein at the cellular surface. A kit for detection the calreticulin at the cellular surface and/or predicting the efficiency of a chemotherapy.

### Background of the invention

Cancer is the major cause of mortality in most industrialized countries. Different ways of cancer treatment can be used : surgery, radiotherapy, immunotherapy, hormonotherapy and chemotherapy. Numerous research laboratories lead works to find cancer therapy improvements. Chemotherapy leads to the cell death. Two type of cell death are known : the apoptosis and the necrosis.

It has long been hypothesized that apoptotic cell death would be poorly immunogenic (or even tolerogenic) whereas necrotic cell death would be truly immunogenic (Bellamy, C.O., Malcomson, R.D., Harrison, D.J. & Wyllie, A.H. Semin Cancer Biol 6, 3-16 (1995) ; Thompson, C.B. Science 267, 1456-1462 (1995) ; lgney, F.H. & Krammer, P.H. Nat Rev Cancer. 2, 277-88 (2002)).

This difference was thought to result from the intrinsic capacity of cells dying from non-apoptotic cell death to stimulate the immune response, for example by stimulating local inflammatory responses ('danger signals') and/or by triggering the maturation of dendritic cells (DCs) (Steinman, R.M., Turley, S., Mellman, I. & Inaba, K. J Exp Med. 191, 411-6 (2000) ; Liu, K. et al. J Exp Med 196, 1091-1097. (2002).

In contrast to necrosis (which is defined by brisk plasma membrane rupture), apoptosis is associated with a series of subtle alterations in the plasma membrane that render the dying cells palatable to phagocytic cells (Kroemer, G. et al. Cell Death Differ 12, 1463-1467 (2005)). Such "eat me" signals, which include the adsorption of soluble proteins from outside the cell (such as C1q and thrombospondin) and the translocation of molecules from inside the cell to the surface (such as phosphatidylserine, PS, and calreticulin, CRT), as well as the suppression of "don't eat me" signals (such as CD47) (Savill, J. & Fadok, V. Nature 407, 784- (2000) ; Lauber, K., Blumenthal, S.G., Waibel, M. & Wesselborg, S. Mol Cell. 14, 277-87 (2004) ; Yoshida, H. et al. Nature 437, 754-8 (2005) ; Gardai, S.J. et al. Cell 123, 321-34 (2005) ; Henson, P.M. & Hume, D.A. Trends Immunol 27, 244-50 (2006)) elicit the recognition and removal of apoptotic cells by professional and non-professional phagocytes. Suboptimal clearance of apoptotic cells can trigger unwarranted immune reactions and lead to autoimmune disease (Hanayama, R. et al. Science 3004, 1147-50. (2004) ; Gaipl, U.S. et al. Curr Top Microbiol Immunol 305, 161-76 (2006)).

Nonetheless, it seems that the dichotomy between immunogenic necrosis versus tolerogenic apoptosis is an oversimplification. Thus, unscheduled (necrotic) tumor cell death may induce local immunosuppression (Vakkila, J. & Lotze, M.T Nat Rev lmmunol 4, 641-8 (2004)). Moreover, the capacity of apoptotic tumor cells to trigger the immune response was found to depend on the apoptosis inducer, leading to the identification of two morphologically undistinguishable subcategories of apoptosis, namely immunogenic versus non-immunogenic apoptosis (Casares, N. et al. J. Exp. Med. 202, 1691-701 (2005) ; Blachere, N.E., Darnell, R.B. & Albert, M.L. PLoS Biol. 3, e185 (2005)).

Most of standard chemotherapies induce a non-immunogenic apoptosis (Zitvogel, L., Casares, N., Pequignot, M., Albert, M.L. & Kroemer, G. Adv. Immunol. 84, 131-79 (2004) ; Steinman, R.M. & Mellman, I. Science 305, 197-200 (2004) ; Lake, R.A. & van der Most, R.G. N Engl J Med 354, 2503-4 (2006)). Thus, even after an initially efficient chemotherapy, patients do not develop an efficient antitumorous immune response and then are overcome by chemotherapy-resistant tumorous variants. To improve anticancer chemotherapy, a promising way is brought by the immunogenic cancer-cell death. Indeed, induction of immunogenic cancer-cell death should be very interesting in that the immune system can contribute through a "bystander effect" to eradicate chemotherapy-resistant cancer cells and cancer stem cells (Steinman, R.M. & Mellman, I. Science 305, 197-200 (2004) ; Lake, R.A. & van der Most, R.G. N Engl J Med 354, 2503-4 (2006); Zitvogel, L., Tesniere, A. & Kroemer, G. Nat Rev Immunol in press (2006)).

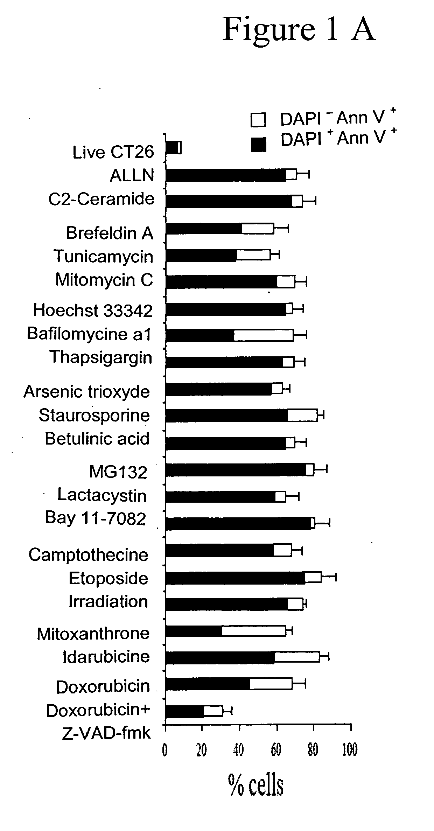

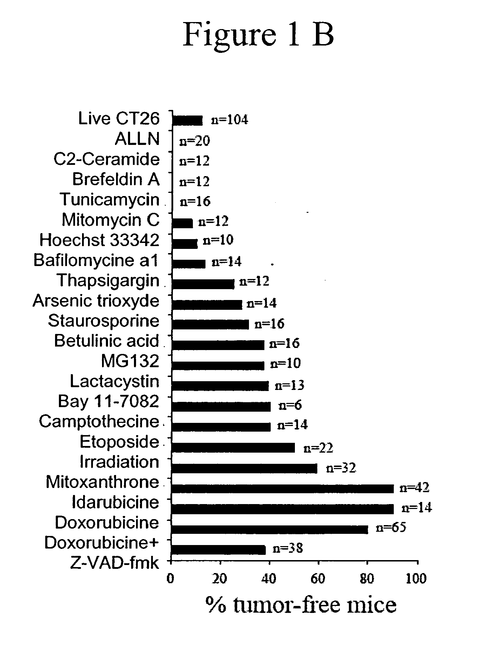

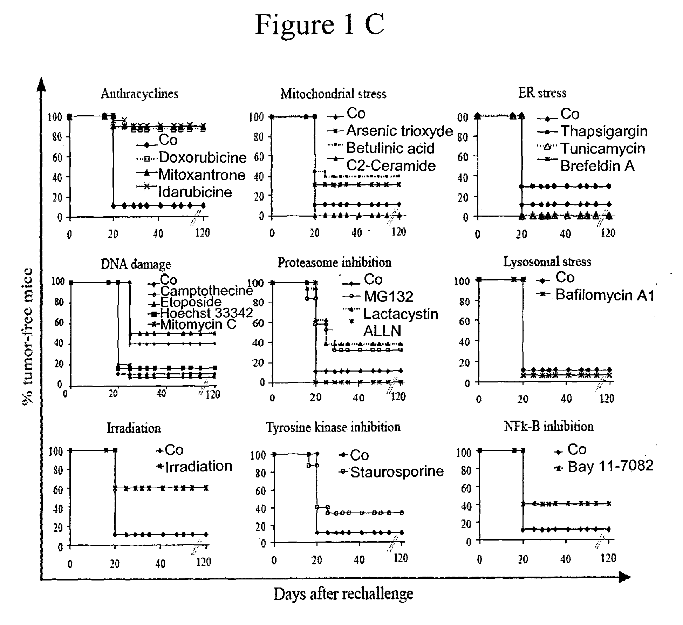

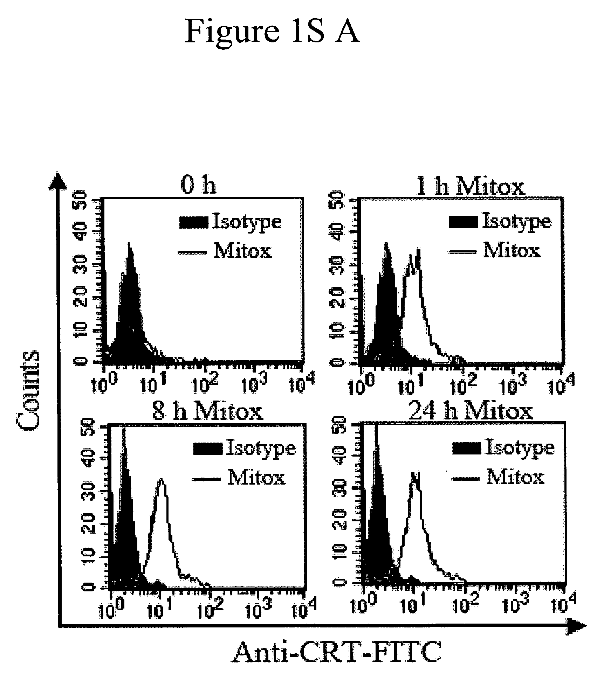

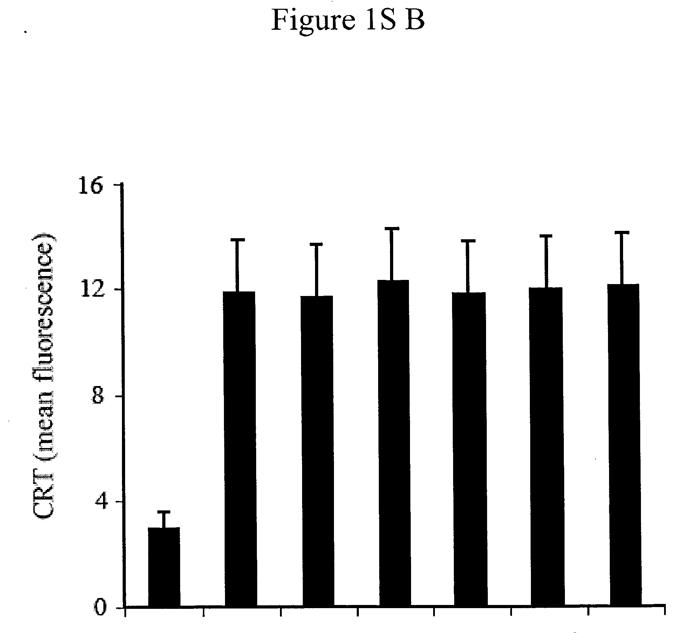

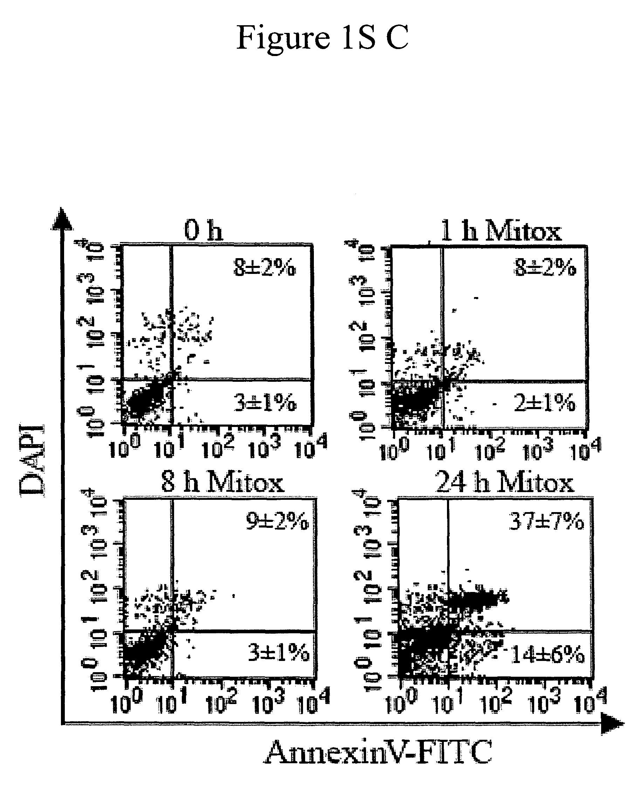

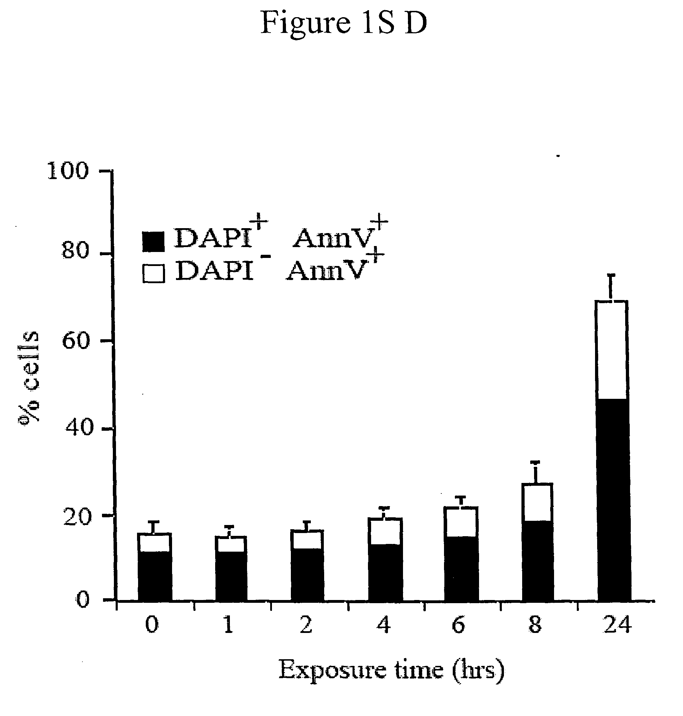

The efficiency of a chemotherapy and the responsiveness is relating to drugs used and the molecules involved in the chemotherapy. The main drugs used in anti-tumorous chemotherapy can be divided in four groups: cytotoxic agents, hormones, immune response modulators and inhibitors of the kinase tyrosin activity. Among cytotoxic agents, we can find cytotoxic antibiotics such anthracyclins (doxorubicin, idarubicin, mitoxantrone which are apoptosis inducers). The inventors have shown for the first time that anthracyclins are capable of eliciting immunogenic apoptosis (Casares, N., Pequignot, M.O., Tesniere, A., Ghiringhelli, F., Roux, S., Chaput, N., Schmitt, E., Hamai, A., Hervas-Stubbs, S., Obeid, M., Coutant, F., Métivier, D., Pichard, E., Aucouturier, P., Pierron, G., Garrido, C., Zitvogel, L., and Kroemer, G. J Exp Med. 202, 1691-701 (2005)). Indeed, while most apoptosis inducers, including agents that target the endoplasmic reticulum (ER) (thapsigargin, tunicamycin, brefeldin), mitochondria (arsenite, betulinic acid, C2 ceramide) or DNA (Hoechst 33343, camptothecin, etoposide, mitomycin C), failed to induce immunogenic apoptosis, anthracyclins elicited immunogenic cell death (as shown in Fig. 1B,C). Despite a growing body of research, under which circumstances an immune response is triggered against dying tumour cells remains an open question (Zitvogel, L., Casares, N., Pequignot, M., Albert, M.L. & Kroemer, G. Adv. Immunol. 84, 131-79 (2004)).

It has been an ongoing conundrum which particular biochemical change would determine the distinction between immunogenic and non-immunogenic cell death.

The present invention is based on the observation that the protein named calreticulin (CRT) exposure is present on cells that succumb to immunogenic cell death, yet lacks on the surface of cells that undergo non-immunogenic cell death.

CRT has been already described for modulating the hormonal response, another way to treat cancer. Proteins which modulate hormone receptor induced gene transcription are present in the nucleus of the cell and inhibit or promote the binding of a hormone to its receptor. The invention described in the US patent US 2003/0060613 A1 presents a purified protein used in modulating hormone responsiveness and efficient in anti-cancer therapy. This invention includes synthetic protein, a mimetic protein, a DNA molecule but also a method of treating a disease such as cancer and a kit containing a pharmaceutical comprising said protein, mimetic of it or synthetic peptide. The protected protein is the CRT which is present either in the endoplasmic reticulum of a cell or in the nucleus. This patent describes mechanism of action on gene transcription and thus protects only nuclear CRT.

Actually, the inventors identified one particular alteration in the plasma membrane of dying cells: the surface exposure of calreticulin (CRT). This event only occurs in immunogenic cancer cell death. Exogenous CRT or external provision of signals that induces CRT exposure confers immunogenicity to otherwise non-immunogenic cell death, allowing for an optimal anti-cancer chemotherapy.

### Disclosure of the invention

Hence, the present invention concerns the calreticulin for its use as a medication for the treatment of a disease in a mammal, said medication inducing an increased location of calreticulin at the cellular surface.

The present invention is based on the identification of CRT exposure as a determining feature of anti-cancer immune responses and delineate a strategy of immunogenic chemotherapy.

The location of the CRT at the cellular surface could be the result of the translocation of intracellular CRT to the cell surface or the result of the translocation of extracellular CRT to the cell surface. So, the present invention concerns the application as a medication wherein the calreticulin (endogenous form or recombinant form or mimetic form) translocation is from the cytoplasm to the membrane of cells or from the extracellular medium to the membrane of cells. As mimetic form, it should be understood a truncated form of the calreticulin or part(s) of calreticulin or hybrids, exhibiting same properties as native form of calreticulin (ie location at the cellular surface).

Furthermore, the inventors have shown that the calreticulin present in an increased amount at the cell surface renders the dying cells palatable to phagocytic cells such as dendritic cells. These cells interact with the immune system and then induce an immune response, that render the calreticulin as an inducer of immunogenic apoptosis. The present invention also concerns calreticulin for its use as a medication for the treatment of a disease in a mammal, said medication inducing an immunogenic apoptosis.

Preferably, by this application as a medication, the disease treated is a cancer such as breast cancer, prostate cancer, melanoma, colon cancer, etc., or an infection like viral or bacterial or fungal or parasitic infection.

The present invention exposes calreticulin for its use as a medication for the treatment of a disease in a mammal, said medication improving the efficiency of chemotherapy in a mammal in need of such chemotherapy by inducing an increased location of CRT at cell surface and/or induction of immunogenic apoptosis.

CRT exposure is known to be induced by UVC light (Gardai, S.J. et al. Cell 123, 321-34 (2005)).

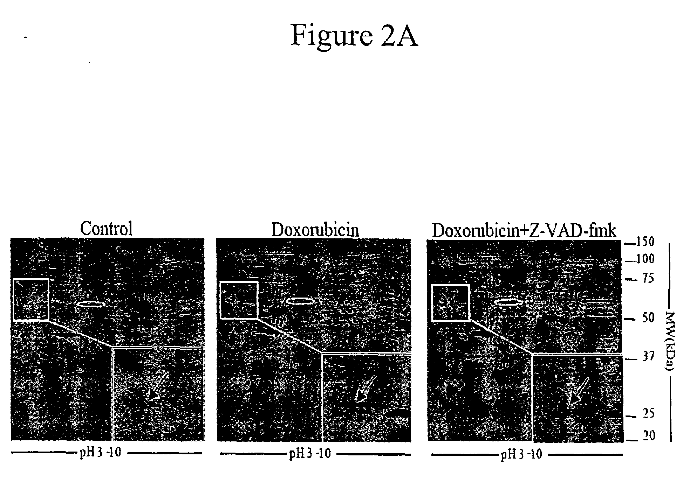

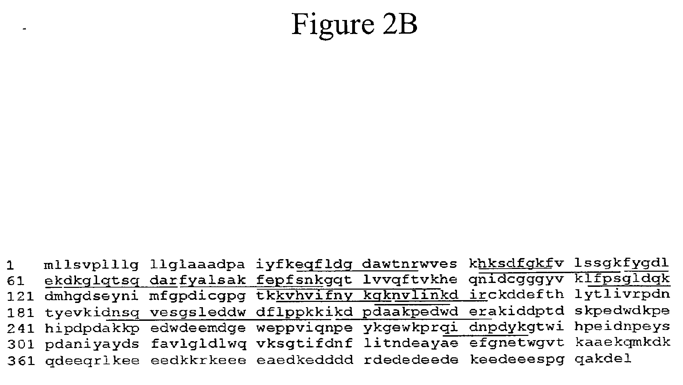

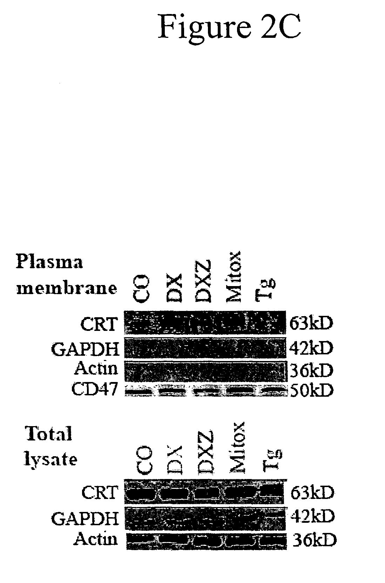

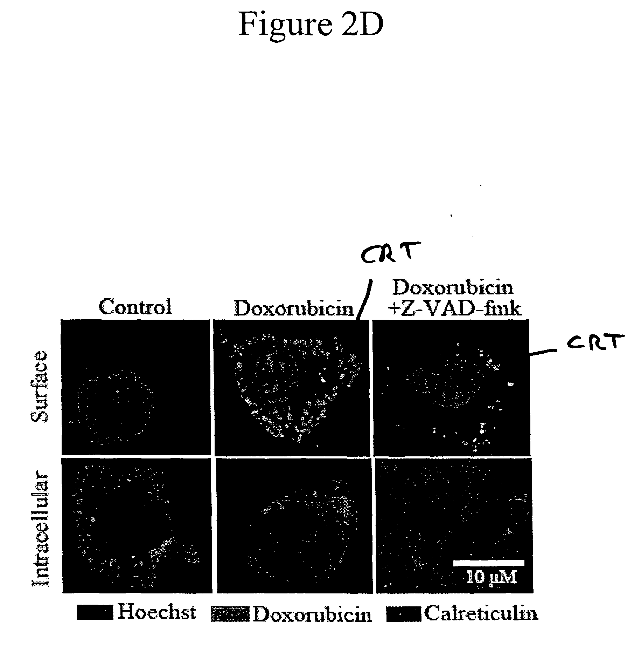

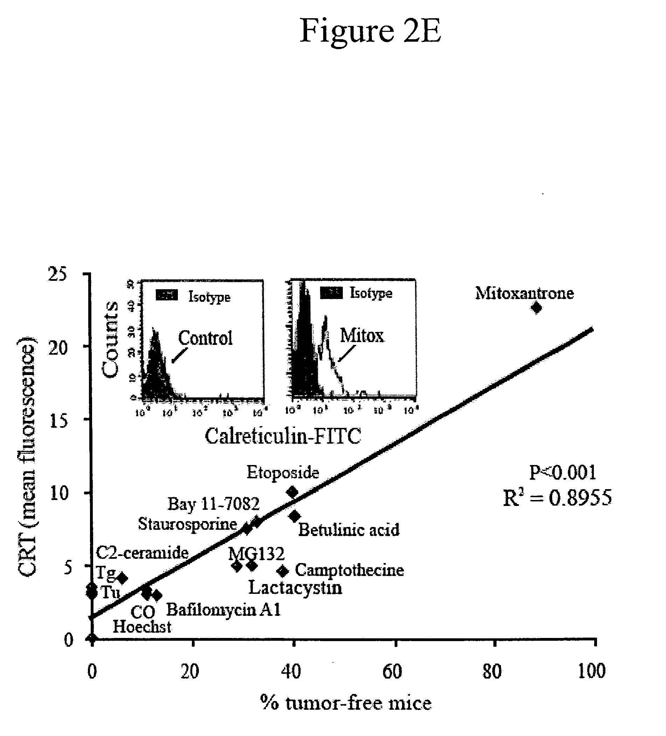

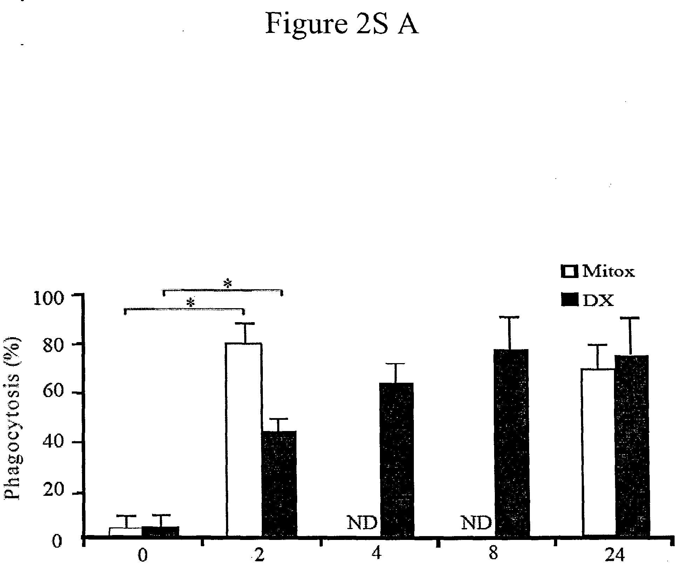

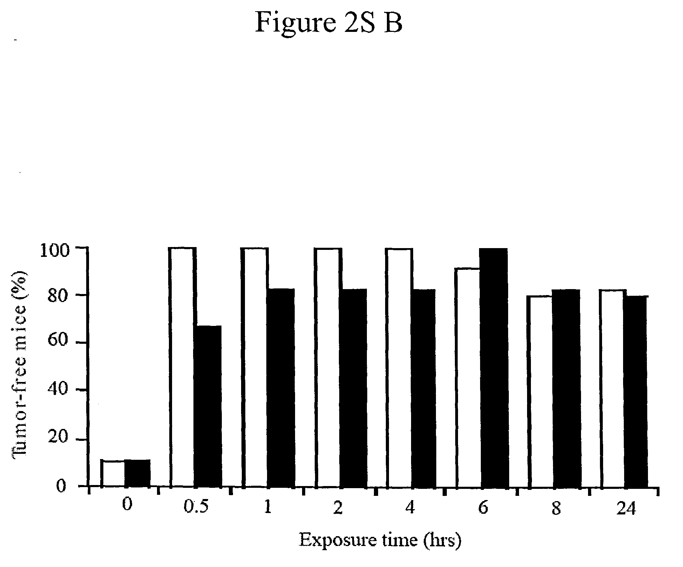

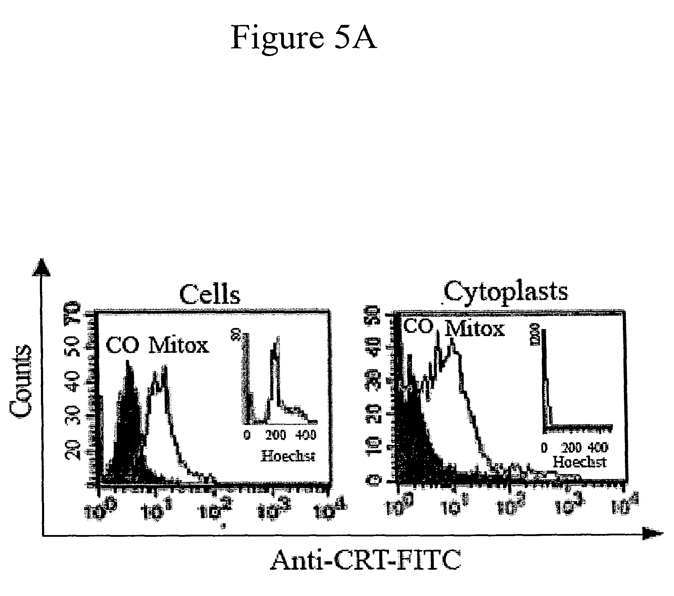

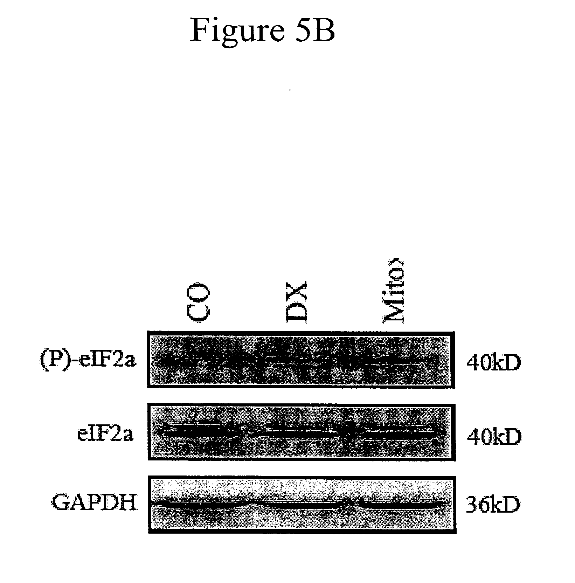

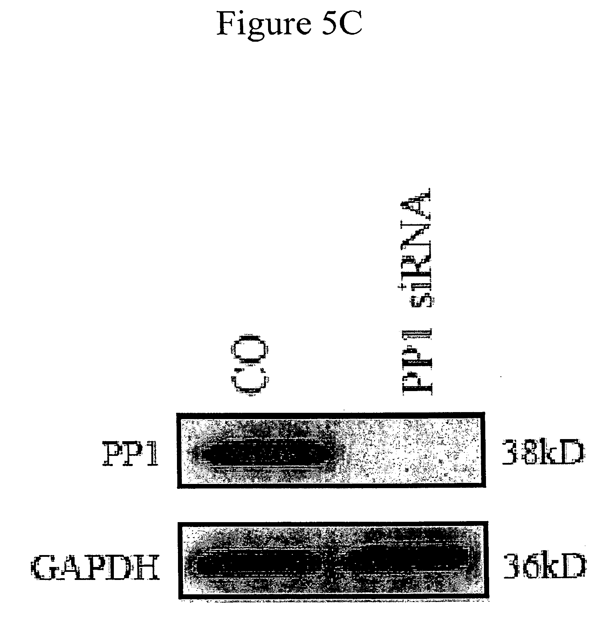

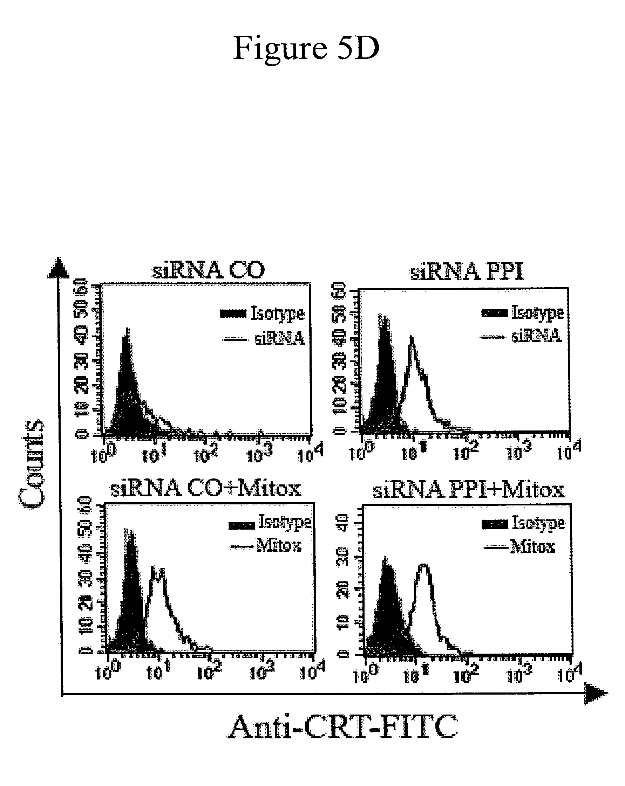

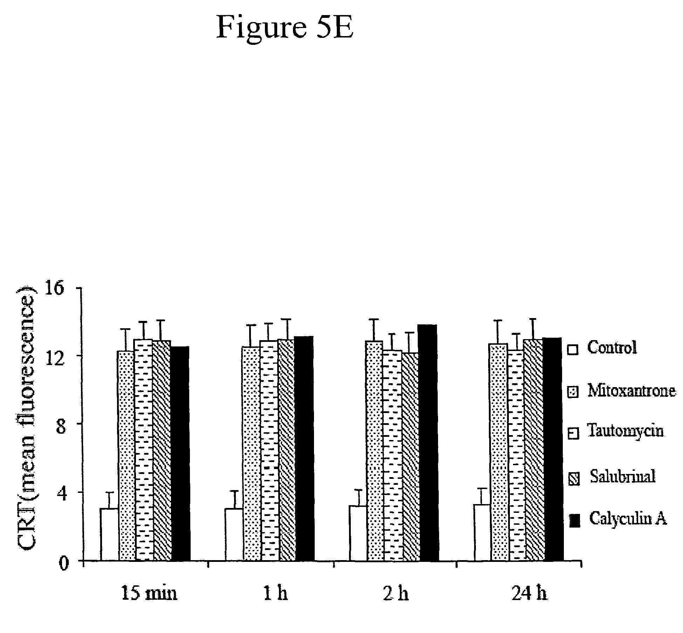

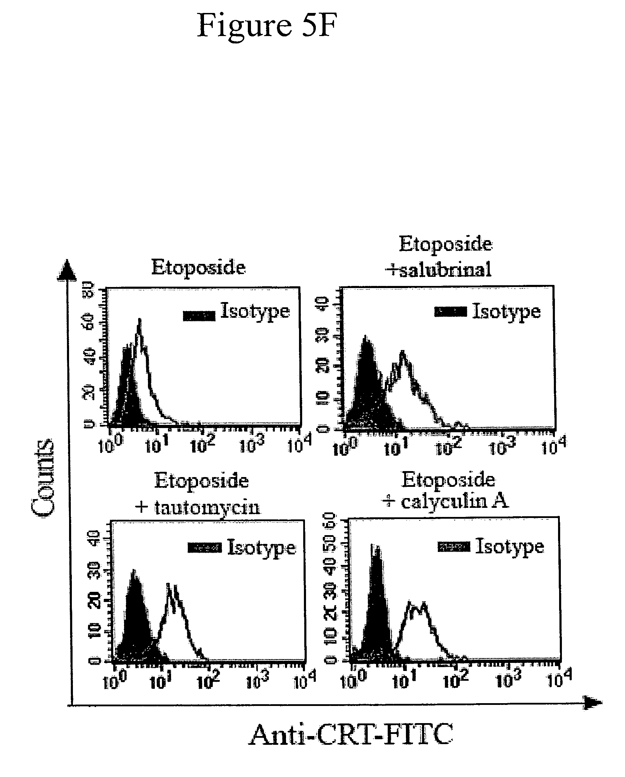

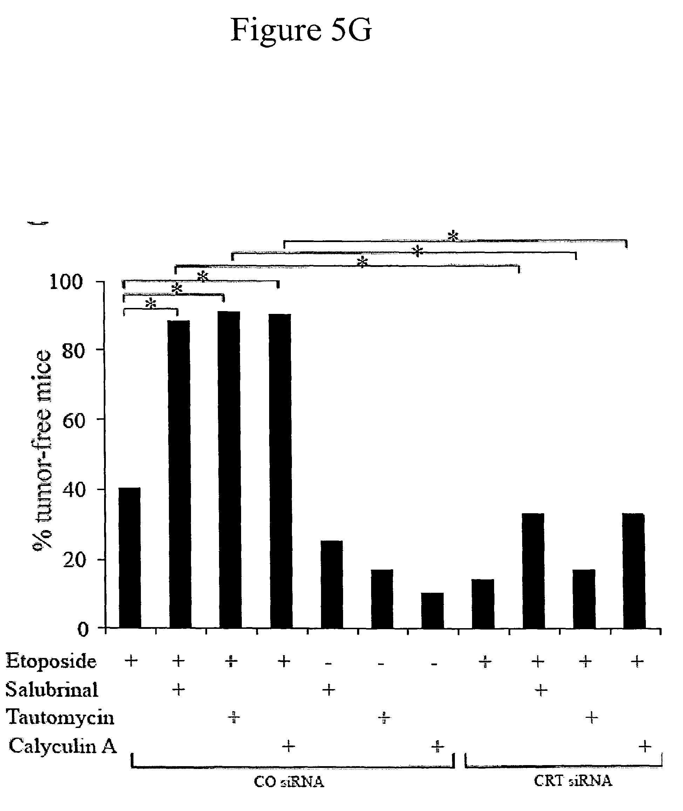

But it appears that CRT exposure is also triggered by anthracyclins (as shown in Fig. 2) and PP1/GADD34 inhibitors (as shown in Fig. 5), involving the translocation of intracellular CRT to the cell surface through a molecular mechanism that is not fully understood and that likewise involves the presence of saturable CRT receptors (Henson, P.M. & Hume, D.A. Trends Immunol 27, 244-50 (2006)) on the cell surface that can bind exogenous CRT as well as endogenous, preformed CRT.

Indeed, the inventors have shown that this CRT protein was strongly (by a factor of 6) induced by doxorubicin and anthracyclin in general (as shown in Fig. 2B and 2C). Immunoblot analyses of 2D gels (not shown) and conventional electrophoreses of purified plasma membrane surface proteins (as shown in Fig. 2C) confirmed the surface exposure of CRT after treatment with anthracyclins. This CRT surface exposure was also detectable by immunofluorescence staining of anthracyclin-treated live cells (as shown in Fig. 2D). The induction of CRT exposure by anthracyclins was a rapid process, detectable as soon as 1 h after treatment (as shown in Fig. 1 S A,B), and hence preceded the apoptosis-associated phosphatidylserine (PS) exposure (as shown in Fig.1S C,D). Of note, there was a strong positive linear correlation (p<0.001) between the appearance of CRT at the cell surface (measured at 4 h) and the immunogenicity elicited by the panel of 20 distinct apoptosis inducers (Fig. 2E).

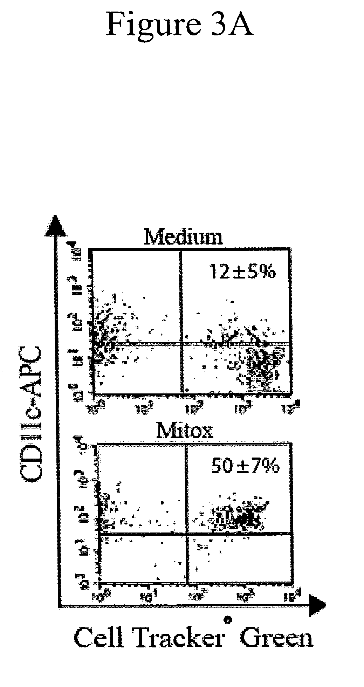

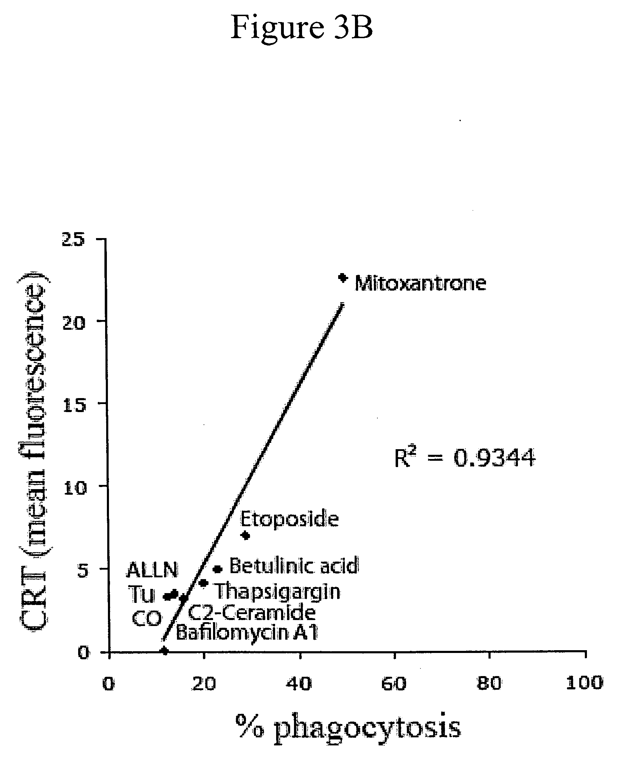

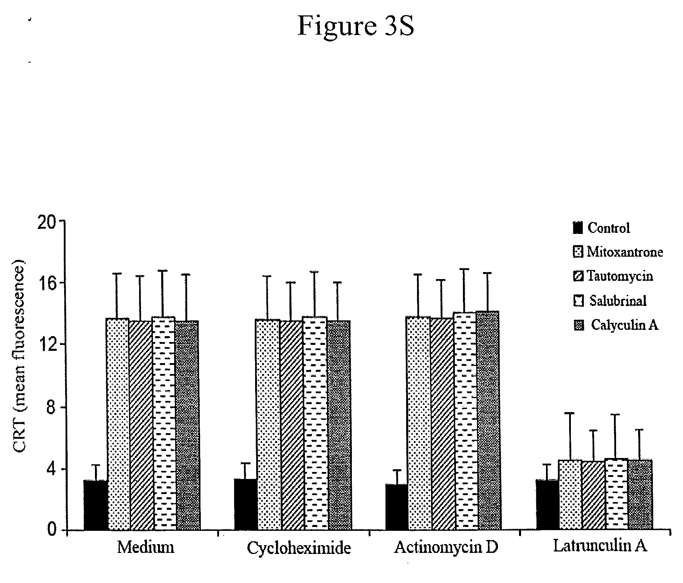

The immunogenicity and the immune response could be mediated by specific cells: dendritic cells (DC). The inventors have shown that anthracyclin-treated tumor cells acquired the property to be phagocytosed by DC few hours after treatment with doxorubicin or mitoxantrone (as shown in Fig. 3A, supplementary Fig. 2A), correlating with the rapid induction of CRT (as shown in Fig.3B, Fig. 1 S A, B) and the acquisition of immunogenicity (as shown in supplementary Fig. 2B).

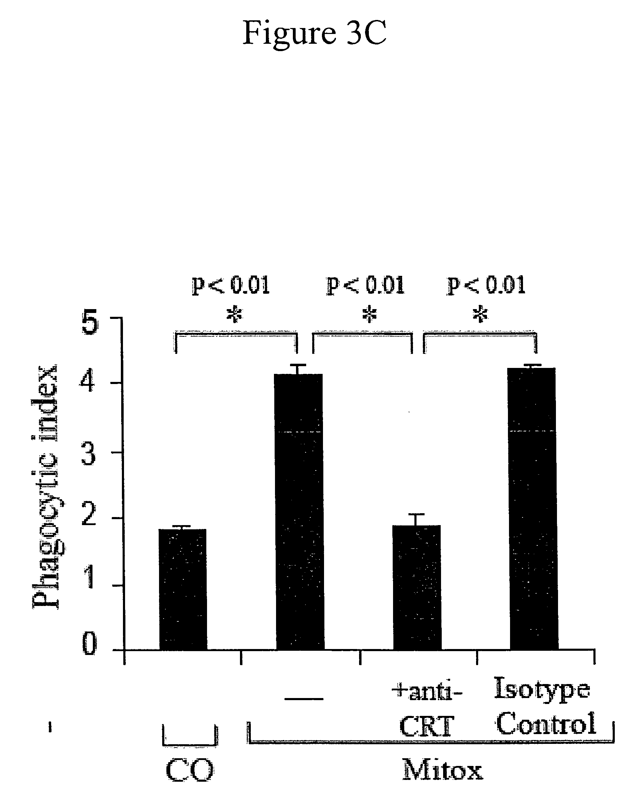

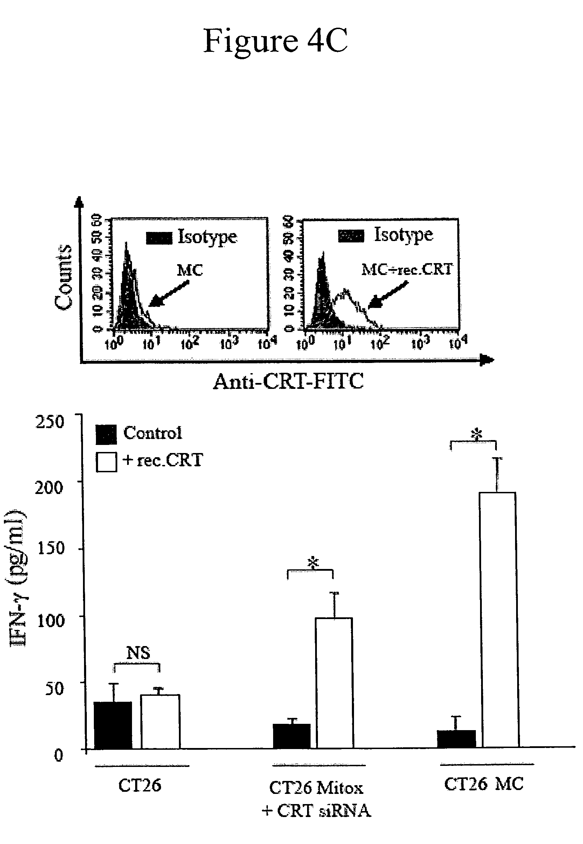

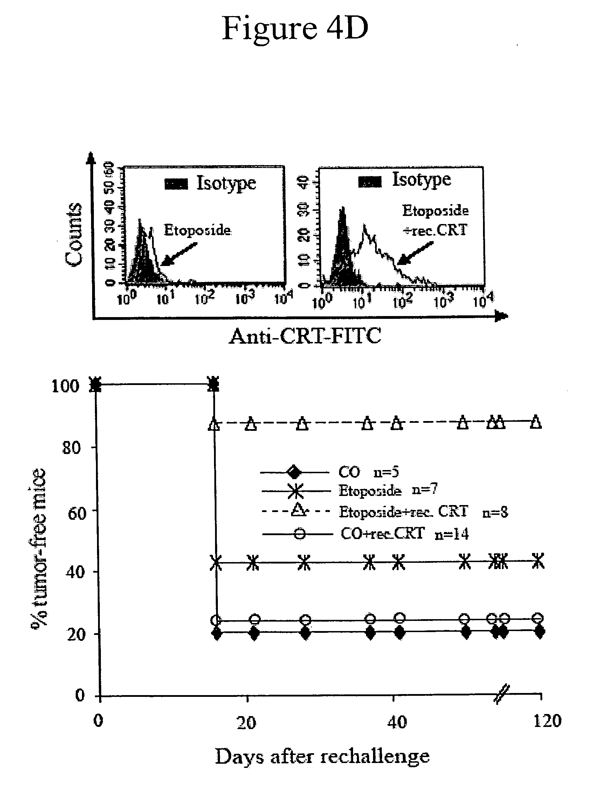

Accordingly, blockade of the CRT present on the surface of mitoxantrone-treated cancer cells by means of a specific antibody inhibited their phagocytosis by DC (as shown in Fig. 3C). Inversely, addition of recombinant CRT protein (rCRT), which binds to the surface of the cells, could reverse the defect induced by the blockade of CRT by antibodies. Hence, surface CRT elicits phagocytosis by DC. Moreover, absorption of rCRT to the plasma membrane surface greatly enhanced the immunogenicity of cells that usually fail to induce an immune response such as mitomycin-treated cells (as shown in Fig. 4C) or etoposide-treated cells coated with rCRT and elicited a vigorous anti-tumor immune response in vivo (as shown in Fig. 4D). However, absorption of rCRT to the cell surface without prior treatment with cell death inducers failed to elicit an anti-cancer immune response. The present invention deals with the recombinant calreticulin for its use as a medication for the treatment of a disease in a mammal, said medication, after the administration of a cell-death inducer (such as etoposide or mitomycine C), inducing an immunogenic apoptosis.

Accordingly, the present invention also concerns the protein phosphatase inhibitor, as the catalytic subunit of the protein phosphatase 1 (PP1) inhibitor, the GADD34 inhibitor or the complex PP1/GADD34 inhibitor, for its use as a medication for the treatment of a disease in a mammal, said inhibitor inducing an increased location of endogenous calreticulin at the cellular surface.

Furthermore, the present invention is directed to the protein phosphatase inhibitor, as the catalytic subunit of the protein phosphatase 1 (PP1) inhibitor, the GADD34 inhibitor or the complex PP1/GADD34 inhibitor, for its use as a medication for the treatment of a disease in a mammal, said inhibitor inducing an immunogenic apoptosis by increased calreticulin translocation at the cellular surface (as shown in Fig. 5).

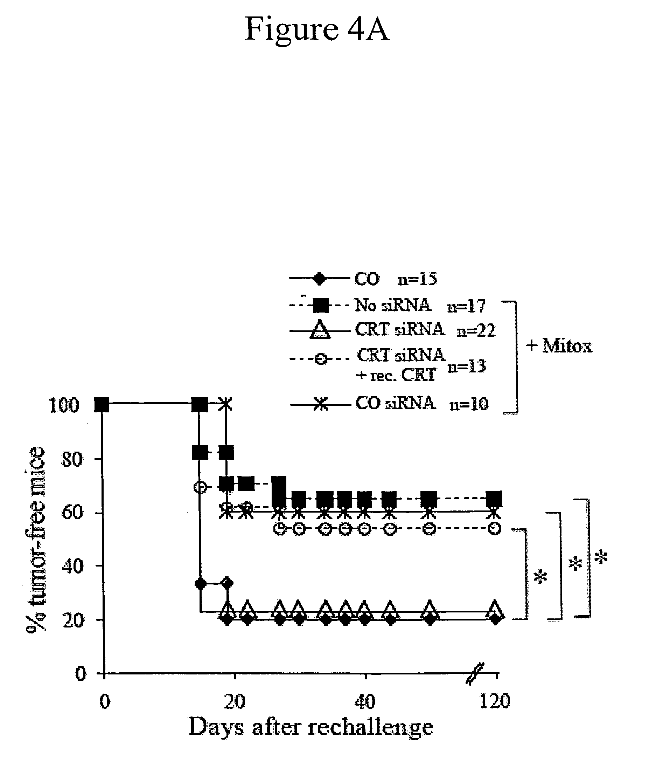

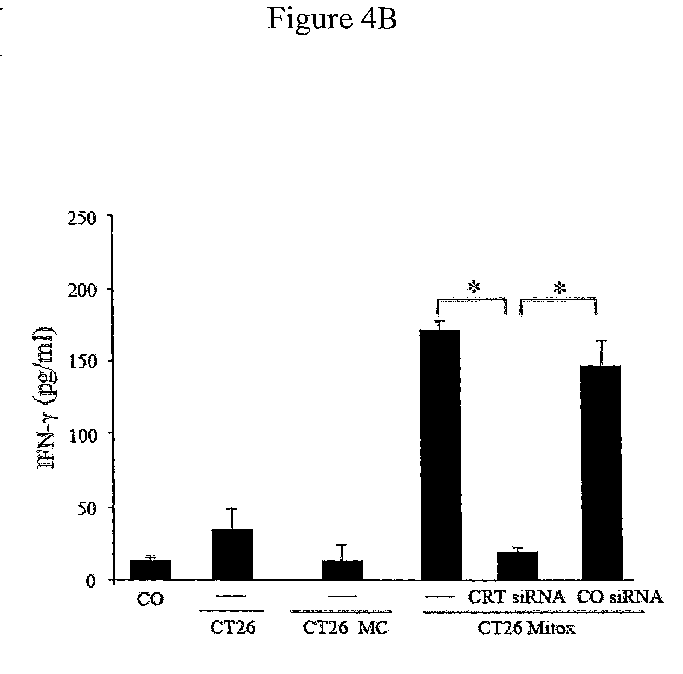

The inventors have observed that CRT exposure induced by anthracyclins and PP1/GADD34 inhibitors was abolished by latrunculin A, an inhibitor of the actin cytoskeleton and exocytose (as shown in supplementary Fig. 4). They have also shown not only that PP1/GADD34 inhibition induces CRT exposure but also that this process can then improve the anti-tumor immune response.

This present invention also concerns the protein phosphatase inhibitor, as the catalytic subunit of the protein phosphatase 1 (PP1) inhibitor, the GADD34 inhibitor or the complex PP1/GADD34 inhibitor, for its use as a medication for the treatment of a disease in a mammal, said inhibitor improving the efficiency of chemotherapy in a mammal in need of such chemotherapy by inducing an increased location of calreticulin at the cellular surface and/or an immunogenic apoptosis

Moreover, an amount of such inhibitor of PP1 or GADD34 or the complex PP1/GADD34 described above can be used in a pharmaceutical composition promoting an increased translocation of the calreticulin protein from the cytoplasm to the cell membrane which thus induces an immune response during apoptosis in a mammal.

Said inhibitor-comprised pharmaceutical composition promoting an increased translocation of the calreticulin from the cytoplasm to the cell surface can also ameliorate chemotherapy response in a mammal.

The inhibitor of PP1 or GADD34 or PP1/GADD34 is advantageously chosen among tautomycin, calyculin A or salubrinal.

On the other hand, eIF2α is a typically hyperphosphorylated in endoplasmic reticulum stress due to the activation of stress kinases (Zhang, K. & Kaufman, R.J. J Biol Chem 279, 25935-8 (2004)). The inventors have observed that kinases, known to phosphorylate eIF2α could be involved in the increased calreticulin translocation and exposure at the cell surface. These kinases are the eukaryotic translation initiation factor 2-alpha kinases, for example heme-regulated inhibitor (HRI, also called Hemin-sensitive initiation factor 2-alpha kinase or eukaryotic translation initiation factor 2-alpha kinase 1), protein kinase RNA activated (PKR, also called eukaryotic translation initiation factor 2-alpha kinase 2), PKR-like ER-localized elF2alpha kinase (PERK, also called eukaryotic translation initiation factor 2-alpha kinase 3) and GCN2 (also called eukaryotic translation initiation factor 2-alpha kinase 4).

The present invention concerns also kinase activator, such as a compound activating one of the eukaryotic translation initiation factor 2-alpha kinases, for example heme-regulated inhibitor (HRI, also called Hemin-sensitive initiation factor 2-alpha kinase or eukaryotic translation initiation factor 2-alpha kinase 1), protein kinase RNA activated (PKR, also called eukaryotic translation initiation factor 2-alpha kinase 2), PKR-like ER-localized elF2alpha kinase (PERK, also called eukaryotic translation initiation factor 2-alpha kinase 3) and GCN2 (also called eukaryotic translation initiation factor 2-alpha kinase 4), for its use as a medication for the treatment of a disease such as a cancer or a viral infection in a mammal, said activator inducing an increased location of endogenous calreticulin at the cellular surface.

The disease treated by such use of medication comprising this activator is a cancer (breast cancer, prostate cancer, melanoma, colon cancer, etc...) or an infection (viral, bacterial, fungal or parasitic infection).

The present invention also provides the application as a medication, comprising at least calreticulin or inhibitor of PP1, GADD34 or PP1/GADD34 or anthracyclin or an activator of said four kinases or anti-calreticulin antibodies or inhibitory/competitive peptide, said medication improves cancer treatment such as tumors sensitive to VP16/etoposide, radiotherapy or immunotherapy ie melanoma, kidney cancer, colon cancer, breast or lung tumors, osteosarcoma.

During autoimmune disorders such as Systemic Lupus Erythematosus (SLE), rheumatoid arthritis, dermatitis..., allergy, graft versus host, transplant rejection,or too-strong-immunogenicity in forced apoptosis, it will be interesting to limit the immunogenicity of cell death. The inventors have observed that this could be obtained by a decreased translocation of calreticulin to the cell surface by the use of blocking or neutralising antibodies anti-calreticulin. An inhibitory or competitive peptide interfering with the translocation of calreticulin could also decrease the amount of calreticulin at the cell surface and then reduce the immunogenicity and the immune response in those diseases.

Thus, the present invention also relates to blocking or neutralizing antibody anti-calreticulin or inhibitory/competitive peptide, interfering with the increased translocation of calreticulin and therefore in the immunogenicity of cell death for its use as a medication for the treatment of autoimmune disorders (SLE, rheumatoid arthritis, dermatitis...), allergy, graft versus host disease, transplant rejection.

In one embodiment of the invention, the anthracyclin as cell death agent can also be used in the preparation of a medication for the treatment of a disease in a mammal, said medication inducing an increased location of calreticulin at the cellular surface.

The anthracyclin can also be used in the preparation of a medication for the treatment of a disease such as cancer or viral infection in a mammal, said medication promoting an induction of immunogenic apoptosis by increased calreticulin translocation at the cellular surface.

The present invention also deals with the use of anthracyclin in the preparation of a medication for the treatment of a disease such as cancer or viral infection in a mammal, said medication improving the efficiency of chemotherapy in a mammal in need of such chemotherapy by inducing an increased location of calreticulin at the cellular surface and/or an immunogenic apoptosis.

Moreover, the present invention concerns also a pharmaceutical composition which comprises an amount of an anthracyclin promoting an increased translocation of the calreticulin protein from the cytoplasm to the cell membrane which thus induces an immune response during apoptosis in a mammal.

Said anthracyclin-comprised pharmaceutical composition promoting an increased translocation of the calreticulin from the cytoplasm to the cell surface can also improve chemotherapy response in a mammal.

The present invention also provides a method promoting the chemotherapy treatment response in a mammal including administration of the pharmaceutical composition comprising an amount of anthracyclin to a mammal in need by inducing an increased location of calreticulin at the cellular surface and/or an immunogenic apoptosis.

The anthracyclin can be doxorubicin, idarubicin or mitoxantrone.

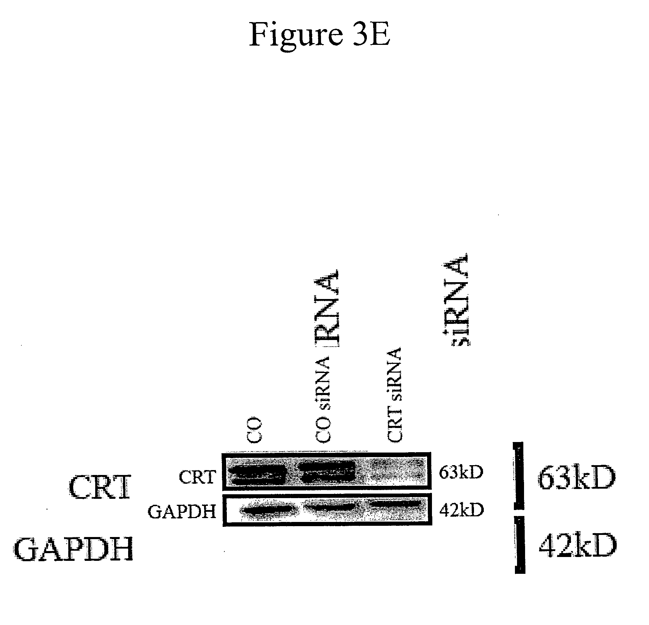

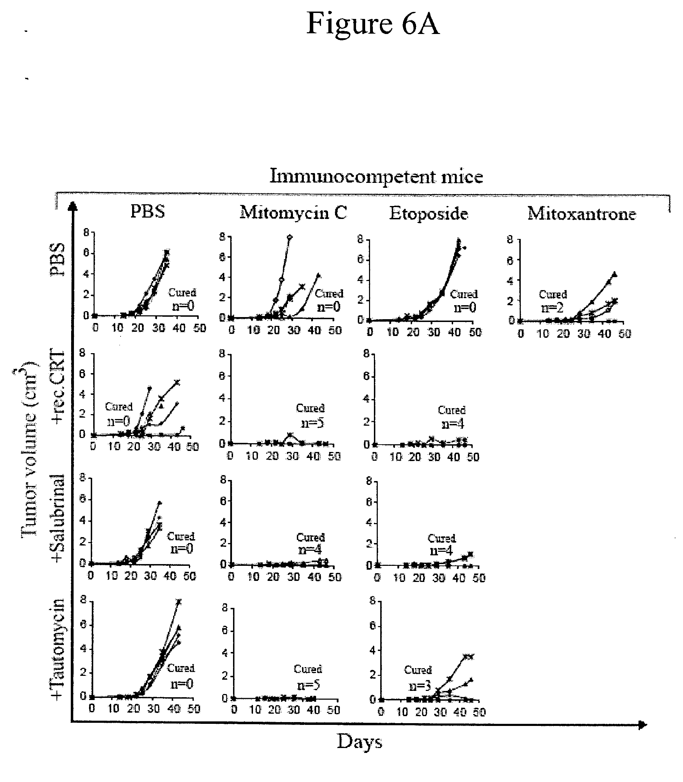

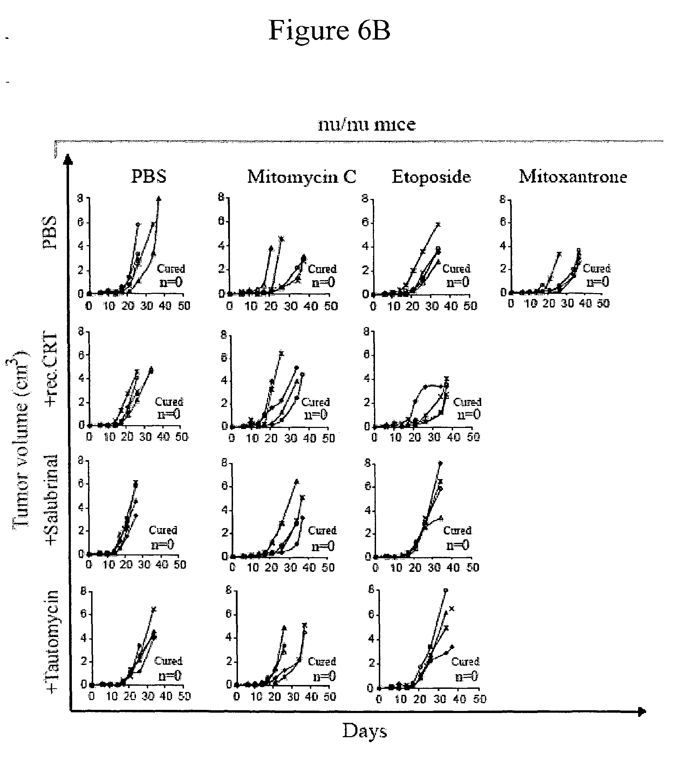

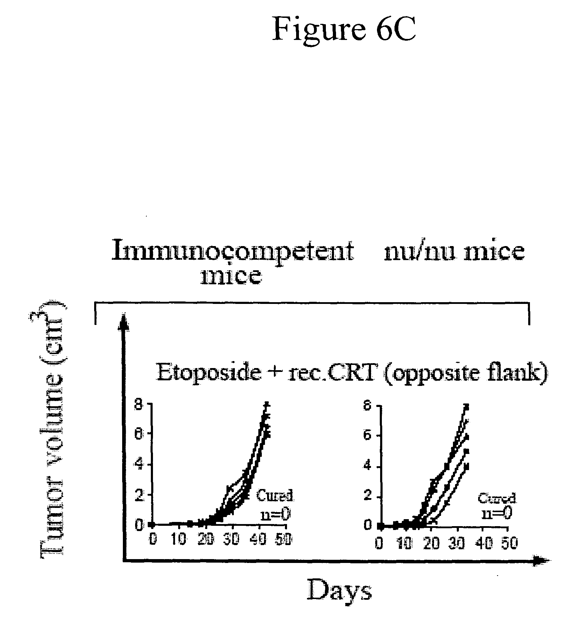

Although CRT adsorbed to the surface of live cells did enhance their phagocytosis by DC in vitro (as shown in Fig 3E), CRT had to be combined with a cell death inducer to elicit a local (as shown in Fig. 4C) or systemic immune response in vivo (as shown in Fig. 4D, Fig. 6). In therapeutics, the inventors have shown that the combination of a cell death inducer (etoposide or mitomycin C) plus calreticulin (rCRT) was able to cause tumor regression, in immunocompetent (but not in immunodeficient) animals. Similarly, etoposide or mitomycin C could be combined with drugs that induce CRT exposure (salubrinal or tautomycin), leading to stable disease or complete tumor regression in immunocompetent (but not in athymic) hosts (as shown in Fig. 6A, B). Live CT26 cells failed to grow in animals that had been cured from CT26 tumors, indicating the establishment of a permanent anti-tumor immune response. As shown here, this knowledge can be employed to stimulate an efficient anti-tumor immune response in which a non-immunogenic chemotherapeutic agent becomes immunogenic when combined with rCRT or PP1/GADD34 inhibitors. These results delineate a strategy of immunogenic chemotherapy for the cure of established cancer such as breast cancer, prostate cancer, melanoma, colon cancer, etc...) or for cure of an infection (viral, bacterial, fungal or parasitic infection).

The present invention also concerns a product containing a chemotherapeutic agent and recombinant calreticulin as a combination product for its use in the treatment of disease.

The present invention also deals with product containing a chemotherapeutic agent and the inhibitors (such as the catalytic subunit of the protein phosphatase 1 (PP1) inhibitor, the GADD34 inhibitor or the complex PP1/GADD34 inhibitor) as a combination product for its use in the treatment of disease. This combination product could be used for the treatment of a disease such as a cancer (breast cancer, prostate cancer, melanoma, colon cancer, etc...) or an infection (viral, bacterial, fungal or parasitic infection).

The chemotherapeutic agent could be etoposide, mitomycin C, anthracyclin and others well known in therapeutics. The present invention also provides a product of combination described just above (chemotherapeutic agent and calreticulin or cell death agent and said inhibitor) wherein said product improves cancer treatment such as tumors sensitive to VP16/etoposide, radiotherapy or immunotherapy ie melanoma, kidney cancer, colon cancer, breast or lung tumors, osteosarcoma.

The present invention would also be directed to a method inducing increased calreticulin translocation from the cytoplasm to the cell surface to enhance an immune response in the apoptosis phenomenon in a mammal, said method comprising administering pharmaceutically effective amount of an inhibitor as the catalytic subunit of the protein phosphatase 1 (PP1) inhibitor, the GADD34 inhibitor or the complex PP1/GADD34 inhibitor.

Differently, the present invention includes also a method inducing increased calreticulin translocation from the cytoplasm to the cell surface to enhance an immune response in the apoptosis phenomenon in a mammal, said method comprising administering pharmaceutically effective amount of an anthracyclin.

The increased calreticulin translocation is preferably from cytoplasm to the membrane of tumorous cells.

This method improves cancer treatment, preferably those tumors sensitive to VP16/etoposide, radiotherapy, or immunotherapy ie melanoma, kidney cancer, colon cancer, breast or lung tumors , osteosarcoma etc.
Preferably, this method is directed to treat chemosensitive cancers as much as immunosensitive cancers.

This method shows increased efficiency of chemotherapy in a mammal in need of such chemotherapy.

Preferably, the mammal treated is a human.

Furthermore, the location of calreticulin protein at the cell surface may be realised by antibodies anti-calreticulin which detect the endogenous form of calreticulin, a recombinant form and the mimetic form. A method of detection of all forms of calreticulin protein at the cellular surface is also an object of the present invention. This could be done *in vitro, ex vivo* or *in vivo.* The methods used to detect this calreticulin protein at the cell surface are well-known from the skilled man of the art. These methods comprise immunochemistry on tissue sections (frozen or paraffined), EIA assays such as ELISA on tumor lysates, confocal immunofluoresence or flow cytometry analyses of cytospins, cell aspirates harvested from tumor beds or autoimmune lesions... One object of the present invention is also to develop a method of quantitative detection of the calreticulin (all forms) at the cellular surface. The immunogenicity of the apoptosis is correlated to the amount of calreticulin present at the cell surface. The more calreticulin at the cellular surface, the more immunogenic apoptosis. This method of detection can serve to predict the immunogenicity of the apoptosis. In addition, the effectiveness of a chemotherapy is correlated to the efficiency of the immune response, therefore the immunogenicity of the apoptosis. The more the immunogenic apoptosis, the more the therapeutic efficiency of a chemotherapy. This method of detection of calreticulin at the cell surface can be used for prediction of immunogenic apoptosis and also for therapeutic efficiency of a chemotherapy. The calreticulin in these methods is used as a predictive marker of both immunogenic apoptosis and therapeutic efficiency of a chemotherapy. This method of quantitative detection can also be advantageous to predict risks of forced apoptosis that becomes too immunogenic. Inhibition of the translocation of the calreticulin at the cellular surface could decrease the immunogenicity of the calreticulin and thus reduce or block (in the best case) the immune response.

There is a basal amount of calreticulin at the cellular surface and the increased amount of calreticulin allows to predict an immunogenic apoptosis and/or a therapeutic efficiency of a chemotherapy. By comparison between before and after chemotherapy, the amount of calreticulin at the cell surface is generally largely increased and the increase will be a good predictive marker for immunogenic apoptosis, therapeutic efficiency of a chemotherapy, immunogenic viral infection but also for autoimmune diseases or transplantation rejection/GVH disease. The present invention also provides a method of detection of the calreticulin at the cell surface wherein the calreticulin at the cell surface is used as a predictive marker of immunogenic viral infection or autoimmune diseases or transplantation rejection/GVH disease.

Additionally, to detect calreticulin, the present invention also provides a kit of detection of the calreticulin protein at the cell surface, according to the method described above, such kit comprising at least anti-calreticulin antibodies. In an embodiment of the invention, this kit of detection could also bean quantitative one for the detection quantitative of calreticulin at the cellular surface, said kit comprising at least antibodies anti-calreticulin.

The present invention also concerns a kit of prediction of immunogenic apoptosis of tumorous cells and/or of a therapeutic efficiency of a chemotherapy, comprising at least anti-calreticulin antibodies for detection of calreticulin protein at the cell surface. When a large amount of calreticulin is detected, the apoptosis of tumorous cells will be immunogenic and the efficiency of the chemotherapy will be ameliorated. A kit of prediction of immunogenic viral infection or autoimmune diseases or transplantation rejection/GVH disease, said kit comprising at least anti-calreticulin antibodies for detection of calreticulin protein at the cellular surface is also provided by the present invention.

The present invention concerns also a method of detection of the calreticulin protein at the cellular surface for the screening of direct or indirect immunogenic drugs and the method of screening for immunogenic drugs including a step of detection of the calreticulin protein at the cell surface, comprising at least anti-calreticulin antibodies for the screening of direct or indirect immunogenic drugs.

The screening of direct and indirect immunogenic drugs could lead to find more efficient anti-tumorous agents. For the direct method, human tumor cell lines must be chosen and then their incubation with a drug panel should allow to dectect if there is a spontaneous CRT translocation from the cytoplasm to the cellular surface by flow cytometry or confocal. For the indirect method of screening, the inventors chose human tumor cell lines where apoptosis is obtained after etoposide treatment but no CRT translocation at the cellular surface. Then, a drug panel is used to detect which one can reverse this phenomenon. This is used to find new inhibitors of the PP1/GADD34 complex or new activators of kinases GCN2, HRI, PERK, PKR.

### Brief description of the drawings

**Fig 1** shows immunogenic cell death induced by anthracyclins.
**Fig.1A.** Frequency of dead and dying cells after treatment with distinct chemotherapeutic agents. CT26 cells were cultured for 24 hours in the presence of the indicated agents for 24-48h, as described in Materials and Methods, and then were stained with Annexin V-FITC and the vital dye DAPI.
**Fig.1B.** Identification of immunogenic cell death inducers.
CT26 cells cultured as in Fig.1A were injected into the left flank, followed by injection of life tumor cells in the right flank 8 days later. The percentage of tumor free mice was determined 120 days later as in Fig.1C.
**Fig.1C.** Incidence of tumors after inoculation of dying cells. The data show the actual frequency of tumor-free mice, for the experiment summarized in Fig. 1 B. Day 1 was considered the day of inoculation of dying tumor cells, 1 week before challenge with dying tumor cells.

**Fig 1S:** Dissociation of CRT exposure and phosphatidyl serine exposure.

**Fig.1SA, Fig.1SB.** Kinetics of CRT exposure. CT26 cells were treated with mitoxantrone for the indicated period, followed by immunofluorescence staining with a CRT-specific antibody and cytofluorometric analysis. Representative pictograms are shown in Fig.1SA and quantitative data are reported in Fig.1SB.
**Fig.1SC, Fig.1SD.** Kinetics of PS exposure and cell death. Cells were cultured as in Fig.1SA and Fig.1SB for the indicated period, followed by staining with Annexin V (which recognizes phosphatidylserin one the surface of dying cells) plus DAPI (which stains dead cells) and FACS analysis.

**Fig 2:** CRT surface exposure in immunogenic cell death.

**Fig.2A-fig.2D.** Identification of CRT as a surface-exposed molecule elicited by anthracyclins. Cells were treated for 4 h with doxorubicin alone (DX) or in combination with Z-VAD-fmk (DXZ), followed by biotinylation of the cell surface and purification of biotinylated proteins, 2D gel electrophoresis (Fig.2A and inserts in Fig.2A showing part of the gel at higher magnification) and mass-spectroscopic identification of one doxorubicin-induced spot as CRT (arrows in Fig.2A and underlined peptides in the CRT protein sequence in Fig.2B), immunoblot detection of CRT in the plasma membrane protein fraction or the total cell lysate (Fig.2C) or immunofluorescence detection of CRT on the cell surface (in non-permeabilized live cells) or within the cell (after permeabilization and fixation) (Fig.2D). Note that the nuclei of untreated cells were visualized with Hoechst 33342 (blue), while those of doxorubicin-treated cells emit a red fluorescence (Fig.2D). Circles in Fig.2A indicate the position of ERP57.
**Fig.2E.** Correlation between CRT exposure and immunogenicity. The surface exposure of CRT was determined by immunoflurorescence cytometry while gating on viable (propidium iodine-negative) cells (inserts) and was correlated with the immunogenicity of cell death (as determined in Fig. 1). CO, control; Tg, thapsigargin; Tu, tunicamycin.

**Fig.2S : Fig.2S A, Fig.2S B:** Kinetics of phagocytosis and immunogenicity elicited by anthracyclins.
CT26 cells were cultured for different periods with mitoxantrone or doxorubicin and then confronted with DC to measure their phagocytosis (Fig.2SA), as in Fig. 3A or injected into mice, one week before challenge with live cells (Fig.2SB). Numbers on each column of Fig.2S B indicate the number of mice that were immunized.

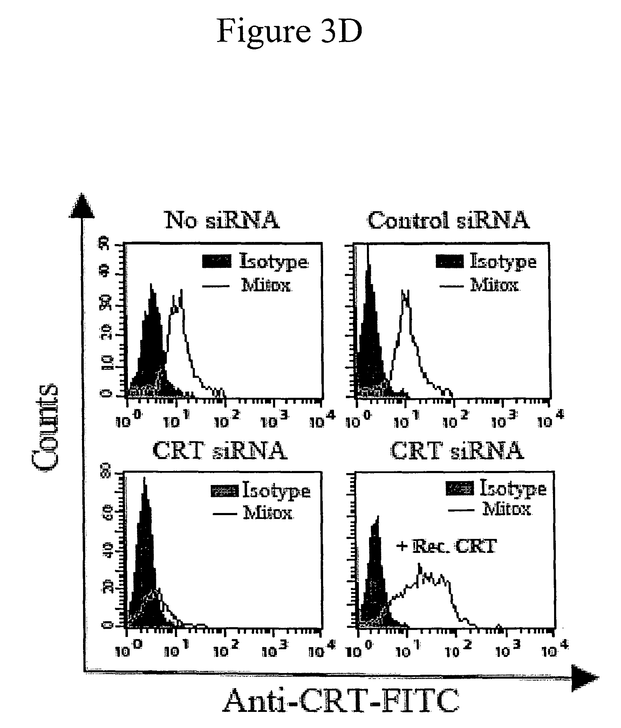

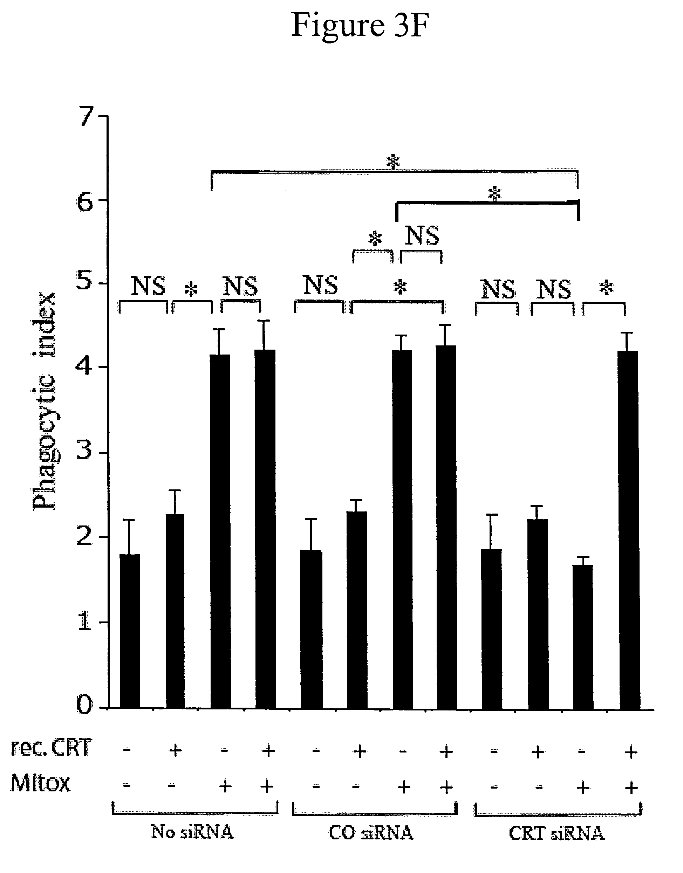

**Fig 3:** Requirement of surface CRT for phagocytosis of tumor cells by DC.

**Fig.3A, Fig.3B.** Correlation between tumor cell phagocytosis and CRT exposure. Tumor cells labeled with Cell Tracker Green were cultured with CD11c-expressing DC and the percentage of DC taking up tumor cells was determined (A) and correlated with the CRT surface exposure (B), measured as in Fig. 2E.
**Fig.3C.** Blockade of CRT inhibits DC-mediated phagocytosis. Mitoxantrone-treated or control cells were incubated with a blocking chicken anti-CRT antibody, followed by detection of phagocytosis by CD.
**Fig.3D, Fig.3E, Fig.3F.** Knock-down of CRT inhibits DC-mediated phagocytosis and rCRT restores phagocytosis. Cells were transfected with the indicated siRNAs and optionally treated with rCRT, followed by immunoblot (Fig.3D) detection of surface CRT (Fig.3E) and phagocytosis by DC (Fig.3F). Results are triplicates (X±SD) and representative of three independent experiments. * denotes statistically significant differences using the Student t' test at p<0.001.

**Fig. 3S : Inhibitory profile of CRT exposure.** Cells were treated with mitoxantrone or inhibitors of PP1/GADD34, after pre-incubation for 1 h with the indicated inhibitors of protein synthesis (cycloheximide), RNA synthesis (actinomycin D), microtubuli (nocodazol), or the actin cytoskeleton (latrunculin A). Then, CRT expression was determined by immunocytofluorometry. Results are means of triplicates ±SD for one representative experiment out of three.

**Fig. 4:** CRT is required for the immune response against dying tumor cells.

**Fig.4A.** In vivo anti-cancer vaccination depends on CRT. CT26 colon cancer cells were transfected with the indicated siRNAs, then treated with rCRT and/or mitoxantrone (as in Fig. 3D) and the anti-tumor response was measured by simultaneously challenging BALB/c mice with mitoxantrone treated tumor cells in one flank and untreated, live tumor cells in the opposite flank.
**Fig.4B.** Priming of T cell responses depending on CRT. CT26 tumor cells were left untransfected or transfected with the indicated siRNAs, then treated with medium alone, mitomycin C or mitoxantrone and injected into the right food pad of Balb/c mice. Five days later, mononuclear cells from the draining popliteal lymph nodes were challenged with freeze-thawed CT26 cells, and IFN-γ secretion was assessed at 72hrs.
**Fig.4C.** Exogenous supply of CRT enhances the immunogenicity of CRT-negative dying cells. CT26 cells lacking CRT expression after depletion of CRT with a siRNA and mitoxantrone treatment or after mitomycin treatment were coated with rCRT (inserts) and then injected int the food pad, followed by assessment of the IFN-γ secretion by cells from the draining lymph nodes as in Fig.4B.
**Fig.4D.** CRT-mediated amelioration of the immune response against etoposide-treated tumor cells. CT26 cells were treated for 24 h with etoposide (or PBS) and rCRT was optionally absorbed to the cell surface (inserts), followed by simultaneous injection of the etoposide ±rCRT-treated tumor cells and live tumor cells in opposite flanks and monitoring of tumor growth.

**Fig. 5.** Induction of calreticulin exposure and immunogenic cell death by inhibition of the PP1/GADD34 complex.

**Fig.5A.** CRT exposure after anthracyclin treatment in the absence of a nucleus. Intact cells or enucleated cells (cytoplasts) were treated for 2 hours with mitoxantrone, followed by immunofluorescence detection of CRT exposure. Inserts show the effective removal of Hoechst 33342-stainable nuclei from the cytoplasts.
**Fig.5B.** Phosphorylation of eIF2α after treatment with anthracyclins. Cells were treated for four hours with mitoxantrone or doxorubicine followed by immunoblot detection of phosphorylated eIF2α irrespective of its phosphorylation state and GAPDH as a loading control.
**Fig5.C-Fig.5D.** Induction of CRT exposure by knock-down of PP1. Cells were transfected with siRNAs specific for the indicated transcripts and were treated 36 h later for 2 h with mitoxantrone prior to immunoblot (Fig.5C) and cell surface staining (Fig.5D).
**Fig.5E-** Kinetics of CRT exposure determine by FACS analysis after incubation of cells with the indicated agents.
**Fig.5F-Fig.5G** PP1/GADD34 inhibitors render cell immunogenic via CRT. Tumor cells were first transfected with a control siRNA or a CRT-specific siRNA and then treated in vitro with etoposide, alone or in combination with PP1/GADD34 inhibitors. Two hours later, the surface CRT was detected to demonstrate the effective expression of CRT on control siRNA-transfected cells treated with etoposide alone or etoposide plus PP1/GADD34 inhibitors (Fig.5F), and later, the cells were injected as in Fig. 1A to determine their capacity to inhibit the growth of live tumor cells inoculated one week later (Fig.5G). The results represent the % of tumor free mice (comprising a total of 12 to 18 mice per group).

**Fig. 6. : Fig.6A, Fig.6B, Fig.6C,** Therapeutic effect of CRT or PP1/GADD34 inhibitors injected into tumors.

CT26 tumors established in immunocompetent wild type (Fig.6A) or athymic *nu*/*nu* Balb/c mice (Fig.6B) were injected locally with the indicated combinations of mitoxantrone, etoposide, mitomycin C, rCRT, salubrinal or tautomycin, followed by monitoring of tumor growth. Each curve represents one mouse. Numbers in the lower right corner of each graph indicate the number of mice that manifest complete tumor involution at day 45. Fig.6C. Identical experimental setting using intratumoral etoposide plus contralateral subcutaneous injection of rec.CRT. The graphs depict one representative experiment out of two, comprising 5 mice/group.

### Detailed description

### Materials and Methods

### Cell lines and cell death induction.

CT26 cells were cultured at 37°C under 5% CO2 in RPMI 1640 medium supplemented with 10% FCS, penicillin, streptomycin, 1 mM pyruvate and 10 mM HEPES in the presence of doxorubicin (DX; 24 h, 25 µM), mitoxantrone (Mitox; 24h, 1µM, Sigma), idarubicin (24h, 1µM, Aventis, France), mitomycin C (30 µM, 48 h; Sanofi-Synthelabo, France), and/or zVAD-fmk (50 µM, 24 h; Bachem), tunicamycin (24h, 65 µM), thapsigargin (24h, 30µM), brefeldin A (24h, 50 µM, Sigma), etoposide (48h, 25µM, Tava classics), MG132 (48h, 10 µM), ALLN (48h, µM), betulinic acid (24h, 10 µM), Hoechst 33343 (24h, 0,2µM), camptothecine (24h, 15 µM), lactacystin (48h, 60 µM), BAY 11-8072 (24h, 30µM), staurosporine (24h, 1.5 µM), bafilomycin A1(48h, 300 nM), arsenic trioxide (24h, 30µM), C2-ceramide (C2-C; 24h, 60 µM,), calyculin A (48h, 30nM), or tautomycin (48h, nM, Sigma) and/or salubrinal (48h, µM, Calbiochem).

### Cell death assays.

Cells were trypsinized and subjected to cytofluorometric analysis with a FACS Vantage after staining with 4,6-diamino-2-phenylindole (DAPI, 2.5 mM, 10 min, Molecular Probes) for determination of cell viability, and Annexin V conjugated with fluorescein isothiocyanate (Bender Medsystems) for the assessment of phosphatidylserine exposure (Zamzami, N. & Kroemer, G. Methods Mol Biol. 282, 103-16 (2004).

### siRNAs and manipulation of surface CRT.

siRNA heteroduplexes specific for CRT (sense strand: 5'-rCrCrGrCUrGrGrGUrCrGrArAUrCrRrArATT-3'), GADD34 5'-(rCrArGrGrArGrCrArGrAUrCrArGrAUrArGrATT-3'), PPICα (5'-rGrCUrGrGrCrCUrAUrArArGrAUrCrArGrATT-3') or an unrelated control (5'rGrCrCrGrGUrAUrGrCrCrGrGUUrArArGUTT-3') were designed in our laboratory and synthesized by Sigma-Proligo. CT26 cells were transfected by siRNAs at a final concentration of 100nM using HiPerFect (Qiagen). Thirty six hours post-transfection CT26 cells were assessed for total CRT content by immunoblotting. To restore CRT expression, cells were exposed to rCRT, produced as described (Culina, S., Lauvau, G., Gubler, B. & van Endert, P.M. Calreticulin promotes folding of functional human leukocyte antigen class I molecules in vitro. J Biol Chem 279, 54210-5 (2004), at 3 µg/10⁶ cells in PBC on ice for 30 min, followed by three washes.

### Fluorescence detection of cell surface CRT.

CT26 cells (on a glass slide or in 12-well plates) were first washed with FACS buffer (1x PBS, 5% fetus bovine serum, and 0.1% sodium azide) and then incubated with rabbit anti-mouse CRT antibody (1:100, Stressgen) in FACS buffer at 4 °C for 30 min. Cells reacted with anti-rabbit lgG (H+L) Alexa fluor 488-conjugates (1:500) in FACS buffer at 4 °C for 30 min. After washing three times with FACS buffer, surface CRT was detected by cytofluorometric analysis on a FACS Vantage. In some experiments, cells were fixed with 4% paraformaldehyde, counterstained with Hoechst (2µM; Sigma), followed by fluorescence microscopic assessment with a Leica IRE2 microscope equipped with a DC300F camera.

### Immunoblot analyses.

Cells were washed with cold PBS at 4°C and lysed in a buffer containing 50 mM Tris HCl pH 6.8, 10 % glycerol and 2 % SDS. Primary antibodies detecting CRT (dilution 1/2000, Stressgen), CD47 (dilution 1/500, BD Biosciences), EIF2∝, EIF2∝-P and PPlc∝ (dilution 1/2000, Cell Signaling Technology), and GADD34 (dilution 1/2000, Abcam), were revealed with the appropriate horseradish peroxidase-labeled secondary antibody (Southern Biotechnologies Associates) and detected by ECL (Pierce). Anti-actin or anti-GAPDH (Chemicon) was used to control equal loading.

### Anti-tumor vaccination and treatment of established tumors.

All animals were maintained in specific pathogen-free conditions and all experiments followed the FELASA guidelines. 3x10⁶ treated CT26 cells were inoculated s.c. in 200 ml of PBS into BALB/c six-week-old female mice (Charles River, France), into the lower flank, while 5x10⁵ untreated control cells were inoculated into the contralateral flank. For the tumorigenicity assay, 3x10⁶ treated or untreated CT26 cells were injected s.c. into *nu*/*nu* mice (IGR animal facility). To assess the specificity of the immune response against CT26, we injected either 5x10⁵ or 5x10⁶ of CT26 (for the mice immunized in a standard protocol or vaccination protocol, respectively). Tumors were evaluated weekly, using a caliper. In a series of experiments, BALB/c (wild type or *nu*/*nu*) carrying palpable CT26 tumors (implanted 14 days before for wild type or 7 days before for nu/nu mice by injection of 10⁶ tumor cells) received a single intratumoral injection of 100 µM PBS containing the same concentration of anti-cancer agents and PP1/GADD34 inhibitors as those used in vitro, as well as rCRT (15 µg). For the assessment of local immune response, 3 x 10⁵ cells were injected in 50 µl into the footpad of mice. Five days later, mice were sacrificed and the draining lymph nodes were harvested. 1x10⁵ lymph node cells were cultured for 4 days alone or with 1x10⁴ CT26 cells killed by a freeze-thaw cycle in 200 µl in round-bottom 96-well plates. IFN-γ was determined by ELISA (BD Pharmingen).

### Generation of BMDCs.

BM cells were flushed from the tibias and femurs of BALB/c mice with culture medium composed of RPMI 1640 medium (Invitrogen Life Technologies) supplemented with 10% heat-inactivated FBS (Invitrogen), sodium pyruvate, 50 µM 2-ME (Sigma), 10 mM HEPES (pH 7.4), and penicillin/streptomycin (Invitrogen). After one centrifugation, BM cells were resuspended in Tris-ammonium chloride for 2 min to lyse RBC. After one more centrifugation, BM cells (1 x 10⁶ cells/ml) were cultured in medium supplemented with 100 ng/ml recombinant mouse FLT3 ligand (R&D systems) in 6-well plates (Costar Corning). After 7 days, the non-adherent and loosely adherent cells were harvested with Versene, washed and transferred in 12-well plates (1.5 x 10⁶ cells/plate) for cocultures with tumor cells.

### Phagocytosis assays.

In 12-well plates, 25 x 10⁶ adherent CT26 cells were labelled with Celltracker Green (Calbiochem) and then incubated with drugs. In some experiments viable CT26 were coated with 2µg/10⁶ cells of chicken anti-CRT antibody (ABR affinity bioreagents) or an isotype control for 30 min prior to washing and feeding to dendritic cells Cs. Alternatively CT26 cells were coated with 2µg/10⁶ cells of rCRT on ice for 30 min and washed twice prior to addition to dendritic cells. Cells were then harvested, washed three times with medium supplemented with FBS and cocultured with immature DC for 2 hours at a ratio of 1:1 and 1:5. At the end of the incubation, cells were harvested with versene, pooled with non-adherent cells present in the supernatant, washed and stained with CD11c-FITC antibody. Phagocytosis was assessed by FACS analysis of double positive cells. Phagocytic indexes refer to the ratio between values obtained at 4°C and values obtained at 37°C of co-incubation between DC and tumor cells.

### Statistical analyses.

Data are presented as arithmetic means ± standard deviation (SD) or percentages. All statistical analyses were performed using JMP software (SAS Institute Inc.). The Student's t-test was used to compare continuous variables (comparison of tumor growth), the Chi square test for non-parametrical variables (comparison of animal cohorts). For all tests, the statistical significance level was set at 0.05.

### Biochemical methods.

The purification of plasma membrane proteins, mass spectroscopy and the generation of cytoplasts are detailed below.

### Biotinylation of CT26 cell surface proteins.

Biotinylation and recovery of cell surface proteins were performed with a method adapted from Gottardi *et al.* (Gottardi, C.J., Dunbar, L.A. & Caplan, M.J. Biotinylation and assessment of membrane polarity: caveats and methodological concerns. Am J Physiol 268, F285-95 (1995)) and Hanwell *et al.*( Hanwell, D., Ishikawa, T., Saleki, R. & Rotin, D. Trafficking and cell surface stability of the epithelial Na+ channel expressed in epithelial Madin-Darby canine kidney cells. J Biol Chem 277, 9772-9 (2002)). Briefly, 20x10⁶ CT26 cells grown on 75 cm² flask were placed on ice and washed three times with ice-cold PBS-Ca²⁺-Mg²⁺(PBS with 0.1 mM CaCl2 and 1 mM MgC12). Membrane proteins were then biotinylated by a 30-min incubation at 4 °C with NHS-SS-biotin 1.25 mg/ml (Pierce) freshly diluted into biotinylation buffer (10 mM triethanolamine, 2 mM CaCl2, 150 mM NaCl, pH 7.5) with gentle agitation. CT26 cells were rinsed with PBS-Ca²⁺-Mg²⁺ + glycine (100 mM) and washed in this buffer for 20 min at 4 °C to quench unreacted biotin. The cells were then rinsed twice with PBS-Ca²⁺-Mg²⁺, scraped in cold PBS, and pelleted at 2,000 rpm at 4 °C. The pellets were solubilized for 45 min in 500µl of lysis buffer (1% Triton X-100, 150 mM NaCl, 5 mM EDTA, 50 mM Tris, pH 7.5) containing protease inhibitors. The lysates were clarified by centrifugation at 14,000 x g for 10 min at 4 °C, and the supernatants were incubated overnight with packed streptavidin-agarose beads to recover biotinylated proteins. The beads were then pelleted by centrifugation, and aliquots of supernatants were taken to represent the unbound, intracellular pool of proteins. Biotinylated proteins were eluted from the beads by heating to 100 °C for 5 min in SDS-PAGE sample buffer before loading onto a 10% SDS-PAGE gel as described above. To ensure the absence of leakage of biotin into the cells, we systematically verified the absence of the intracellular protein actin and GAPDH in biotinylated extracts.

### 2D gel electrophoresis analysis and protein identification by mass spectrometry.

Purified proteins were precipitated using the Ettan 2-D clean up kit (GE Healthcare) were subsequently resuspended in urea buffer (7M urea, 2M thiourea, 2% Chaps, 1% Sulfobetaine SB3-10, 1% Amidosulfobetaine ASB14, 50mM DTT). For the first dimension of protein separation, isoelectric focusing (IEF) was performed using 18-cm immobilized nonlinear pH gradient strips (pH 3 to 10; GE Healthcare) on a IPGphor II electrophoresis unit (GE Healthcare). Proteins (100 µg) were loaded by in-gel rehydratation for 9h, using low voltage (30V) then run using a program in which the voltage was set for 1 h at 100 V, 2 h at 200 V, 1 h at 500 V, 1 h at 1,000 V, 2hrs, 2hrs voltage gradient 1,000-8,000V and 4 h at 8,000 V. Prior to the second-dimension electrophoresis, IPG gel strips were equilibrated for 10 min at room temperature in 1% dithiothreitol to reduce the proteins and sulfhydryl groups were subsequently derivatized using 4% iodoacetamide (both solutions were prepared in 50 mM Tris [pH 8.8]-6 M urea-30% glycerol-2% SDS-2% bromophenol blue). Strips were transferred to 1.0-mm-thick 10% (wt/vol) polyacrylamide gels (20 by 20 cm), and the second-dimension gels were run at 50 µA for 6 h. Gels were stained with Sypro Ruby (BioRad) and visualized using a Typhoon 9200 scanner (GE Healthcare) .The Investigator HT analyser (Genomic Solutions Inc) was used for matching and analysis of visualized protein spots among differential gels. Background subtraction was used to normalize the intensity value representing the amount of protein per spot.

Differentially expressed spots were excised from the gels with an automatic spot picker (Investigator ProPic, Genomic Solutions Inc.), placed in Eppendorf tubes, and destained by washing for 5 min with 50 µL of 0.1 M NH4HCO3. Then 50 µL of 100% acetonitrile were added incubated for other 5 min. The liquid was discarded, the washing steps were repeated one more time and gel plugs were shrunk by addition of pure acetonitrile. The dried gel pieces were reswollen with 4.0 ng/µL trypsin (Promega, Madison, WI) in 50 mM NH4HCO3 and digested overnight at 37°C. Peptides were concentrated with ZipTip®µC18 pipette tips. Co-elution was performed directly onto a MALDI target with 1 µL of α-cyano-4-hydroxycinnamic acid matrix (5 µg/mL in 50% acetonitrile, 0.1% TFA). MALDI-MS and MALDI-MS/MS were performed on an Applied Biosystems 4700 Proteomics Analyzer with TOF/TOF ion optics. Spectra were acquired in positive MS reflector mode and calibrated either externally using five peaks of standard (ABl4700 Calibration Mixture) or internally using porcine trypsin autolysis peptide peaks (842.51, 1045.56 and 2211.10 [M+H]⁺ ions). Mass spectra were obtained from each sample spot by 30 sub-spectra accumulation (each consisting of 50 laser shots) in a 750 to 4000 mass range. Five signal-to-noise best peaks of each spectrum were selected for MS/MS analysis. For MS/MS spectra, the collision energy was 1 keV and the collision gas was air (Medzihradszky, K.F. et al. The characteristics of peptide collision-induced dissociation using a high-performance MALDI-TOF/TOF tandem mass spectrometer. Anal Chem 72, 552-8 (2000)).

MS and MS/MS data were interpreted using the GPS Explorer software (Version 2.1, Applied Biosystems) which acts as an interface between the Oracle database containing raw spectra and a local copy of the MASCOT search engine (Version 1.8). Peptide mass fingerprints obtained from MS analysis were used for protein identification in Swiss Prot non-redundant database. All peptide mass values are considered monoisotopic and mass tolerance was set <50 ppm. Trypsin was given as the digestion enzyme, 1 missed cleavage site was allowed, methionine was assumed to be partially oxidized and serine, threonine and tyrosine partially phosphorylated. Mascot (Matrix Science) scores greater than 71 were considered to be significant (p<0.005). For MS/MS analysis, all peaks with a signal-to-noise ratio greater than 5 were searched against the Swiss Prot database using the same modifications as the MS database. Fragment tolerance less than 0.3 Da was considered.

### Preparation of cytoplasts.

Trypsinized CT26 cells were enucleated as described in the publication of Andreau, K. et al. Contagious apoptosis facilitated by the HIV-1 envelope. Fusion-induced cell-to-cell transmission of a lethal signal. J. Cell Sci. 117, 5643-53 (2004). Briefly, cells were treated in 2 ml of complete RPMI medium containing cytochalasin B (10µg/ml; Sigma) and DNase I (80U/ml ; Sigma). Cell suspension was adjusted to a final concentration of 5x10⁶/ml and incubated at 37°C for 45 minutes before being layered onto a previously prepared discontinuous Ficoll (Pharmacia) density gradient (3 ml of 100%, in 1 ml of 90% and 3 ml of 55% Ficoll Paque layer containing 5µg/ml cytochalasin B and 40U/ml DNase I ; gradients were prepared in ultracentrifuge tubes and pre-equilibrated at 37°C in a CO2 incubator overnight). Gradients containing cell suspensions were centrifugated in a prewarmed SW41 Beckman rotor at 25 000 rpm for 20 minutes at 30°C. The cytoplasts-enriched fraction was collected from the interface between 90 and 100% Ficoll layers, washed in complete RPMI medium, and incubated at 37°C. The cells were incubated with MTX, CA, Sal and TA for the period of time indicated in the experiment. Then the cell surface CRT was detected (see materials and methods) and the viability was determined by with propidium iodine staining (2µg/ml, Sigma) for 5 min followed by cytofluorometric analysis. Alternatively cythoplasts were cocultured with immature DC for 2 hours at a ratio of 1:1 and 1:5. At the end of the incubation, cells were harvested with versene, pooled with non-adherent cells present in the supernatant, washed and stained with CD11c-FITC antibody. Phagocytosis was assessed by FACS analysis of double positive cells.

Different examples are given in order to complete and illustrate the invention of the present application.

### Example1: CRT exposure defines immunogenic cell death.

Dying CT26 tumor cells exposed to a panel of -20 distinct apoptosis inducers (all of which induced ~70±10% apoptosis, as determined by double staining with the vital dye DAPI and the PS-binding dye Annexin V, Fig. 1A) were injected into one flank of immunocompetent BALB/c mice, followed by rechallenge of the animals with live tumor cells injected into the opposite flank 8 days later. Protection against tumor growth then was interpreted as a sign of anti-tumor vaccination (Fig. 1 B) because such protection was not observed in athymic (*nu*/*nu*) BALB/c mice (Casares, N. et al. J. Exp. Med. 202, 1691-701 (2005).and data not shown). Most apoptosis inducers, including agents that target the endoplasmic reticulum (ER) (thapsigargin, tunicamycin, brefeldin), mitochondria (arsenite, betulinic acid, C2 ceramide) or DNA (Hoechst 33342, camptothecin, etoposide, mitomycin C), failed to induce immunogenic apoptosis, while anthracyclins (doxorubicin, idarubicin, mitoxantrone) elicited immunogenic cell death (Fig. 1B,C). To identify changes in the plasma membrane proteome, we affinity-purified biotinylated surface proteins from cells that were either untreated or short-term (4 h) treated with doxorubicin or doxorubicin plus Z-VAD-fmk, a pan-caspase inhibitor that reduces the immunogenicity of doxorubicin-elicited cell death (Casares, N. et al. J. Exp. Med. 202, 1691-701 (2005).and Fig. 1 B). Comparison of 2D electrophoreses (Fig. 2A), followed by mass spectroscopic analyses, led to the identification of CRT (Fig. 2B) as a protein that was strongly (by a factor of 6) induced by doxorubicin, but less so (by a factor of 1.8) by doxorubicin combined with Z-VAD-fmk. Another protein whose surface exposure was specifically induced by doxorubicin were identified as ERP57 (Fig. 2A), a CRT-interacting chaperone (Bedard, K., Szabo, E., Michalak, M. & Opas, M. Int Rev Cytol 245, 91-121 (2005). Immunoblot analyses of 2D gels (not shown) and conventional electrophoreses of purified plasma membrane surface proteins (Fig. 2C) confirmed the surface exposure of CRT after treatment with anthracyclins. This CRT surface exposure was also detectable by immunofluorescence staining of anthracyclin-treated live cells (Fig. 2D) and was not accompanied by a general increase in the abundance of intracellular CRT (Fig. 2C,D). The induction of CRT exposure by anthracyclins was a rapid process, detectable as soon as 1 h after treatment (Fig. 1 S A,B), and hence preceded the apoptosis-associated phosphatidylserine (PS) exposure (Fig. 1S C,D). CRT exposure did not correlate with alterations in CD47 expression (Fig. 2C). Of note, there was a strong positive linear correlation (p<0.001) between the appearance of CRT at the cell surface (measured at 4 h) and the immunogenicity elicited by the panel of 20 distinct apoptosis inducers (Fig. 2E).

### Example 2: Requirement of CRT for DC-mediated recognition of dying tumor cells.

In view of the established role of CRT as an "eat me" signal (Gardai, S.J. et al. Cell 123, 321-34 (2005); Ogden, C.A. et al. J Exp Med 194, 781-95 (2001)), we decided to further investigate the possible implication of CRT in the phagocytosis of anthracyclin-treated tumor cells by DC, a cell type that is stringently required for mounting an immune response against apoptotic tumor cells (Steinman, R.M., Turley, S., Mellman, I. & Inaba, K. J Exp Med. 191, 411-6 (2000); Casares, N. et al. J. Exp. Med. 202, 1691-701 (2005)). Anthracyclin-treated tumor cells acquired the property to be phagocytosed by DC quickly, well before the manifestation of apoptotic changes, within a few hours after treatment with doxorubicin or mitoxantrone (Fig. 3A, Fig. 2S A), correlating with the rapid induction of CRT (Fig. 3B, Fig. 1 S A, B) and the acquisition of immunogenicity (Fig. 2S B). The presence of CRT on the surface of tumor cells treated with a panel of distinct cell death inducers strongly correlated with their DC-mediated phagocytosis, suggesting that CRT is important in mediating the uptake of tumor cells by DC (Fig. 3B). Accordingly, blockade of the CRT present on the surface of mitoxantrone-treated cancer cells by means of a specific antibody from avian origin (which cannot interact with mouse Fc receptors) inhibited their phagocytosis by DC (Fig. 3C). Similarly, knockdown of CRT with a specific siRNA (Fig. 3D, E) suppressed the phagocytosis of anthracyclin-treated tumor cells (Fig. 2F). Addition of recombinant CRT protein (rCRT), which binds to the surface of the cells, could reverse the defect induced by the CRT-specific siRNA, both at the level of CRT expression (Fig. 3E) and phagocytosis by DC (Fig. 3F). Of note, rCRT alone could not promote DC maturation ex vivo over a large range of concentrations. Hence, surface CRT elicits phagocytosis by DC.

### Example 3: Requirement of CRT for immunogencity of dying tumor cells.

The knock-down of CRT compromised the immunogenicity of mitoxantrone-treated CT26 cells, and this defect was restored when rCRT was used to complement the CRT defect induced by the CRT-specific siRNA. This result was obtained in two distinct experimental systems, namely (i) when CT26 tumor cells were injected into the flank of Balb/c mice (or MCA205 cells were injected into C57BI/6 mice, not shown) to assess the efficacy of anti-tumor vaccination (Fig 4A) and (ii) when the tumor cells were injected into the foot pad to measure interferon-γ production by T cells from the popliteal lymph node (Fig. 4B). In this latter system, absorption of rCRT to the plasma membrane surface greatly enhanced the immunogenicity of cells that usually fail to induce an immune response such as mitomycin-treated cells (Fig. 4C). Similarly, etoposide-treated cells coated with rCRT elicited a vigorous anti-tumor immune response in vivo, in conditions in which sham-coated cells treated with etoposide were poorly immunogenic (Fig. 4E). However, absorption of rCRT to the cell surface without prior treatment with cell death inducers failed to elicit an anti-cancer immune response and live rCRT-pretreated cells inoculated into mice formed tumors, both in immunocompetent (Fig. 4E) and immunodeficient mice (not shown). Thus, CRT critically determines the immunogenicity of cell death in vivo but does not determine cell death as such.

### Exampe 4: Inhibitors of PP1/GADD34 induce CRT exposure and induce immunogenicity.

Since anthracyclin-induced CRT exposure was a rather rapid process (within 1 h, Fig. 1SA, 1SB), we suspected that anthracyclins might exert effects that are not mediated by genotoxic stress. In response to mitoxantrone, enucleated cells (cytoplasts) readily (within 1 h) exposed CRT (Fig. 5A) and became preys of DC (not shown) as efficiently as intact cells (Fig. 3A), indicating the existence of a cytoplasmic (non-nuclear) anthracyclin target. Anthracyclins failed to induce immediate mitochondrial stress (not shown), yet caused the rapid phosphorylation of eIF2α (Fig. 5B), a protein that is typically hyperphosphorylated in ER stress due to the activation of stress kinases (Zhang, K. & Kaufman, R.J. J Biol Chem 279, 25935-8 (2004). Knock-down of the four kinases known to phosphorylate elF2α (GCN2, HRI, PERK, PKR) failed to inhibit the anthracyclin-stimulated CRT exposure (not shown). In contrast, knock-down of either GADD34 or the catalytic subunit of protein phosphatase 1 (PP1) (Fig. 5C), which together form the PP1/GADD34 complex involved in the dephosphorylation of elF2αwas sufficient to induce CRT exposure (Fig. 5D and not shown). The CRT exposure triggered by PP1 or GADD34 depletion was not further enhanced by mitoxantrone (Fig. 5D), suggesting that PP1/GADD34 and anthracyclins act on the same pathway to elicit CRT translocation to the cell surface. CRT exposure was efficiently induced by chemical PP1/GADD34 inhibitors, namely tautomycin, calyculin A (which both inhibit the catalytic subunit of PP1)(Gupta, V., Ogawa, A.K., Du, X., Houk, K.N. & Armstrong, R.W. J Med Chem 40, 3199-206 (1997)), as well as by salubrinal (which inhibits the PP1/GADD34 complex)(Boyce, M. et al. Science 307, 935-9 (2005)) (Fig. 5E). All these PP1/GADD34 inhibitors induced CRT exposure with a similar rapid kinetics as did anthracyclins, both in cells (Fig. 5E) and in cytoplasts. Mitoxantrone and salubrinal induced CRT exposure on a panel of tumor cell lines from murine (MCA205, B16F10, J558) or human origin (HeLa, A549, HCT116). CRT exposure induced by anthracyclins and PP1/GADD34 inhibitors was not affected by inhibitors of transcription, translation or microtubuli, yet was abolished by latrunculin A, an inhibitor of the actin cytoskeleton and exocytosis (Fig. 3S). Inhibition of the PP1/GADD34 complex with salubrinal, calyculin A or tautomycin was not sufficient to induce immunogenic cell death (Fig. 5F, G) (and the cells, which did not die, formed lethal tumors when injected into animals). However, these inhibitors greatly enhanced However, these inhibitors greatly enhanced CRT exposure (Fig. 5F) and the immunogenic potential of cells succumbing to etoposide (Fig. 5G) or mitomycin C (not shown) and this immunostimulatory effect was abrogated by knocking down CRT (Fig. 5G). Altogether, these results demonstrate that PP1/GADD34 inhibition induces CRT exposure, which in turn can stimulate the anti-tumor immune response.

### Example 5: Immunogenic chemotherapy by in vivo application of CRT or PP1/GADD34 inhibitors.

A single intratumoral injection of mitoxantrone into established 14-day-old CT26 tumors was able to cause their permanent regression in some but not all cases, if the tumors were established in immunocompetent BALB/c mice (Fig. 6A). However, there was no cure by mitoxantrone if the tumors were carried by immunodeficient *nu*/*nu* mice (Fig. 6B). The intratumoral injection of rCRT, salubrinal, tautomycin, etoposide or mitomycin C had no major therapeutic effect, neither in immuncompetent nor in *nu*/*nu* mice. However, the combination of a cell death inducer (etoposide or mitomycin C) plus rCRT was able to cause tumor regression, in immunocompetent (but not in immunodeficient) animals. To obtain a therapeutic effect, rCRT had to be injected into the tumor. rCRT injected into a distant site did not ameliorate the antitumoral effects of intratumorally injected etoposide (Fig. 6C).Similarly, etoposide or mitomycin C could be combined with drugs that induce CRT exposure (salubrinal or tautomycin), leading to stable disease or complete tumor regression in immunocompetent (but not in athymic) hosts (Fig. 6A, B). Live CT26 cells failed to grow in animals that had been cured from CT26 tumors, indicating the establishment of a permanent anti-tumor immune response. Similar results were obtained when established MCA205 sarcomas (in C57BI/6 mice) or PRO colon carcinomas (in BDIX rats) were treated by local injections of weakly immunogenic cell death inducers plus rCRT or PP1/GADD34 inhibitors (not shown). These results delineate a strategy of immunogenic chemotherapy for the cure of established cancer.

## Claims

1. Calreticulin for its use as a medication for the treatment of a disease in a mammal, said medication inducing an increased location of calreticulin at the cellular surface.

2. Calreticulin for its use as a medication for the treatment of a disease in a mammal, said medication inducing an immunogenic apoptosis.

3. Calreticulin according to claim 1 or 2, for its use as a medication for the treatment of a disease in a mammal, said medication improving the efficiency of chemotherapy in a mammal in need of such chemotherapy.

4. Recombinant calreticulin for its use as a medication for the treatment of a disease in a mammal, said medication, after the administration of a cell-death inducer (such as etoposide or mitomycine C), inducing an immunogenic apoptosis.

5. Protein phosphatase inhibitor, as the catalytic subunit of the protein phosphatase 1 (PP1) inhibitor, the GADD34 inhibitor or the complex PP1/GADD34 inhibitor, for its use as a medication for the treatment of a disease in a mammal, said inhibitor inducing an increased location of endogenous calreticulin at the cellular surface.

6. Protein phosphatase inhibitor, as the catalytic subunit of the protein phosphatase 1 (PP1) inhibitor, the GADD34 inhibitor or the complex PP1/GADD34 inhibitor, for its use as a medication for the treatment of a disease in a mammal, said inhibitor inducing an immunogenic apoptosis.

7. Protein phosphatase inhibitor, as the catalytic subunit of the protein phosphatase 1 (PP1) inhibitor, the GADD34 inhibitor or the complex PP1/GADD34 inhibitor, for its use as a medication for the treatment of a disease in a mammal, said inhibitor improving the efficiency of chemotherapy in a mammal in need of such chemotherapy.

8. Protein phosphatase inhibitor as claimed in one of the claim 5 to 7, wherein the inhibitor is tautomycin, calyculin A or salubrinal.

9. The use of anthracycline in the preparation of a medication for the treatment of a cancer in a mammal, said medication inducing a location of calreticulin at the cellular surface.

10. The use of anthracycline in the preparation of a medication for the treatment of a viral infection in a mammal, said medication inducing a location of calreticulin at the cellular surface.

11. The use of anthracycline in the preparation of a medication for the treatment of a cancer in a mammal, said medication promoting an induction of immunogenic apoptosis by calreticulin translocation at the cellular surface.

12. The use of anthracycline in the preparation of a medication for the treatment of a viral infection in a mammal, said medication promoting an induction of immunogenic apoptosis by calreticulin translocation at the cellular surface.

13. The use of anthracyclin according to claim 9 to 12, in the preparation of a medication for the treatment of a disease in a mammal, said medication improving the efficiency of chemotherapy in a mammal in need of such chemotherapy.

14. Use according to claim 9 to 13, wherein the anthracyclin is doxorubicin, idarubicin or mitoxantrone.

15. Kinase activator, such as a compound activating one of the eukaryotic translation initiation factor 2-alpha kinases, for example heme-regulated inhibitor (HRI, also called Hemin-sensitive initiation factor 2-alpha kinase or eukaryotic translation initiation factor 2-alpha kinase 1), protein kinase RNA activated (PKR, also called eukaryotic translation initiation factor 2-alpha kinase 2), PKR-like ER-localized elF2alpha kinase (PERK, also called eukaryotic translation initiation factor 2-alpha kinase 3) and GCN2 (also called eukaryotic translation initiation factor 2-alpha kinase 4), for its use as a medication for the treatment of a disease such as a cancer or a viral infection in a mammal, said activator inducing an increased location of endogenous calreticulin at the cellular surface.

16. Blocking or neutralizing antibody anti-calreticulin or inhibitory/competitive peptide, interfering with the increased translocation of calreticulin and therefore in the immunogenicity of cell death for its use as a medication for the treatment of autoimmune disorders (such as SLE, rheumatoid arthritis, dermatitis), allergy, graft versus host disease, transplant rejection.

17. The application as a medication according to one of the claim 1 to 16, wherein the calreticulin (endogenous, recombinant and mimetic form) translocation is from the cytoplasm to the membrane of cells or from the extracellular medium to the membrane of cells.

18. The application as a medication according to one of the claim 1 to 17, wherein the disease treated is a cancer (such as breast cancer, prostate cancer, melanoma, colon cancer) or an infection (viral, bacterial, fungal or parasitic infection).

19. The application as a medication according to one of the claim 1 to 18, said application of the medication improves cancer treatment such as tumors sensitive to VP16/etoposide, radiotherapy or immunotherapy ie melanoma, kidney cancer, colon cancer, breast or lung tumors, osteosarcoma.

20. A pharmaceutical composition which comprises an amount of an inhibitor as the catalytic subunit of the protein phosphatase 1 (PP1) inhibitor, the GADD34 inhibitor or the complex PP1/GADD34 inhibitor promoting an increased translocation of the endogenous calreticulin protein from the cytoplasm to the cellular surface which thus induces an immune response during apoptosis in a mammal.

21. A pharmaceutical composition which comprises an amount of an inhibitor as the catalytic subunit of the protein phosphatase 1 (PP1) inhibitor, the GADD34 inhibitor or the complex PP1/GADD34 inhibitor promoting an increased translocation of the endogenous calreticulin protein from the cytoplasm to the cellular surface which thus improves chemotherapy response in a mammal.

22. A pharmaceutical composition which comprises an amount of an anthracyclin, like doxorubicin, idarubicin or mitoxantrone, promoting an increased translocation of the endogenous calreticulin protein from the cytoplasm to the cell membrane which thus induces an immune response during apoptosis in a mammal.

23. A pharmaceutical composition which comprises an amount of an anthracyclin, like doxorubicin, idarubicin or mitoxantrone, promoting an increased translocation of the endogenous calreticulin protein from the cytoplasm to the cell membrane which thus improves chemotherapy response in a mammal.

24. A method promoting the chemotherapy treatment response in a mammal comprising administration of the pharmaceutical composition of claim 20 to 23 to a mammal in need.

25. Product containing a chemotherapeutic agent and recombinant calreticulin as a combination product for its use in the treatment of disease.

26. Product containing a chemotherapeutic agent and the inhibitors according to claim 5 as a combination product for its use in the treatment of disease.

27. Product of one of the claims 25 or 26, wherein the disease treated is the cancer such as breast cancer, prostate cancer, melanoma, colon cancer) or an infection (viral, bacterial, fungal or parasitic infection).

28. Product of one of the claims 25 or 26, wherein said product improves cancer treatment such as tumors sensitive to VP16/etoposide, radiotherapy or immunotherapy ie melanoma, kidney cancer, colon cancer, breast or lung tumors, osteosarcoma.

29. A method of detection of the calreticulin protein as the endogenous form, a recombinant form or a mimetic form, at the cellular surface.

30. Method according to claim 29, wherein the calreticulin at the cellular surface is used as a predictive marker of an immunogenic apoptosis.

31. Method according to claim 29, wherein the calreticulin at the cell surface is used as a predictive marker of the therapeutic efficiency of a chemotherapy.

32. Method according to claim 29, wherein the calreticulin at the cell surface is used as a predictive marker of immunogenic viral infection or autoimmune diseases or transplantation rejection/GVH disease.

33. Method according to claim 29, **characterised in that** the said protein detection is realised by different methods such as immunohistochemistry on tissue sections, EIA assays such as ELISA on tumor lysates, confocal immunofluoresence or flow cytometry analyses of cytospins, cell aspirates harvested from tumor beds or autoimmune lesions.

34. Method of detection of the calreticulin protein at the cellular surface for the screening of direct or indirect immunogenic drugs.

35. Kit of detection of the calreticulin protein at the cell surface according to the method of claim 29, comprising at least anti-calreticulin antibodies.

36. Kit of prediction of immunogenic apoptosis of tumorous cells comprising at least anti-calreticulin antibodies for detection of calreticulin protein at the cell surface.

37. Kit of prediction of therapeutic efficiency of a chemotherapy comprising at least anti-calreticulin antibodies for detection of calreticulin protein at the cellular surface.

38. Kit of prediction of immunogenic viral infection or autoimmune diseases or transplantation rejection/GVH disease comprising at least anti-calreticulin antibodies for detection of calreticulin protein at the cellular surface.

39. Method of screening for immunogenic drugs including a step of detection of the calreticulin protein at the cell surface according to the method of claim 29, comprising at least anti-calreticulin antibodies for the screening of direct or indirect immunogenic drugs.
